(19) Europäisches Patentamt
European Patent Office
Office européen des brevets

(11) Publication number: 0 187 702
B1

(12) EUROPEAN PATENT SPECIFICATION

(45) Date of publication of patent specification: 14.03.90

(21) Application number: 86300037.8

(22) Date of filing: 06.01.86

(51) Int. Cl.[5]: C 07 C 213/00, C 07 D 295/08, C 07 D 453/02, B 01 F 17/18, A 61 K 9/14

(54) N-(omega,omega-1-dialkoxy)- and N-(omega,omega-1-dialkenoxy)-alk-1-yl-N,N,N-trisubstituted ammonium surfactants, their preparation and pharmaceutical formulations containing them.

(30) Priority: 07.01.85 US 689407

(43) Date of publication of application:
16.07.86 Bulletin 86/29

(45) Publication of the grant of the patent:
14.03.90 Bulletin 90/11

(84) Designated Contracting States:
AT BE CH DE FR GB IT LI LU NL SE

(56) References cited:
US-A-3 567 729

ANNUAL REVIEW OF BIOPHYSICS AND BIOENGINEERING, vol. 7, 1978, Palo Alto R.E. PAGANO et al. "Interactions of liposomes with mammalian cells" pages 435-468

(73) Proprietor: SYNTEX (U.S.A.) INC.
3401 Hillview Avenue
Palo Alto California 94303 (US)

(72) Inventor: Eppstein, Deborah Anne
2290 Louis Road
Los Altos California 94303 (US)
Inventor: Jones, Gordon Henry
21550 Edward Way
Cupertino California 95014 (US)
Inventor: Felgner, Philips Louis
1080 Fremont Avenue
Los Altos California 94022 (US)
Inventor: Roman, Richard Bolton
104 Pecan Street
Fairhope Alabama 36532 (US)

(74) Representative: Armitage, Ian Michael et al
MEWBURN ELLIS & CO. 2/3 Cursitor Street
London EC4A 1BQ (GB)

EP 0 187 702 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to glycerol-based cationic compounds.

The compounds of this invention are illustrated by formula I

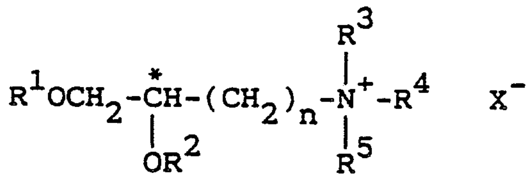

I

or an optical isomer thereof wherein $R^1$ and $R^2$ are the same or different and are alkyl or alkenyl of 6 to 22 carbons; $R^3$, $R^4$ and $R^5$ may be the same or different and are hydrogen, alkyl of 1 to 8 carbons, aryl, aralkyl of 7 to 11 carbons, or when two or three of $R^3$, $R^4$, and $R^5$ are taken together form quinuclidino, piperidino, pyrrolidino, or morpholino; n is 1 to 8; and X is a pharmaceutically acceptable anion.

These compounds may be used in any of the many uses for which cationic suractants find application. For example, they may be used in industrial application, in food or feeds, in pharmaceutical formulation, cosmetological compositions, or other areas where surfactants may be employed.

As these compounds are pharmaceutically non-toxic compounds, they may also be used in cosmetology, for example, in makeups, lipstick, eyeshadow material, they may also be used in fingernail polishes, in body lotions, moisturizing creams, and the like. These compounds may also be used for application to the hair, either alone or in combination with other materials, such as in shampoos, hair conditioners, permanent wave formulations or hair straighteners, or as components in hair creams, gels, and the like.

Of particular interest is the use of these compounds in pharmaceutical formulations, particularly topical formulations such as ointments, gels, pastes, creams, and the like; and more particularly for the preparation of pharmaceutical formulations containing liposomes. The consistency of the formulation depends on the amount of aqueous solution used to make the formulation. In such formulations containing compounds of this invention, drugs which are insoluble or only sparingly soluble themselves in aqueous solutions can be solubilized so that a greater concentration of drug can be presented to the body.

In pharmaceutical formulations, these compounds may be used in those contexts where cationic surfactants are acceptable for the formulation of creams, pastes, gels, colloidal dispersions, and the like. For additional information, reference is made to *Remington's Pharmaceutical Sciences,* 16th Edition, Mack Publishing Company, Easton, Pennsylvania (1980), editor Arthur Osol, or any other standard treatise on pharmaceutical formulations.

The compounds of this invention are particularly useful in the preparation of liposomes. Liposomes are microscopic vesicles (or blisters) consisting of concentric lipid bilayers, generally spherically-shaped. Structurally, liposomes range in size and shapes from long tubes to spheres, with dimensions of a few hundred Angströms to fractions of a millimeter. Regardless of the overall shape, the bilayers are generally organized as closed concentric lamellae, with an aqueous layer separating each lamella from its neighbor. Vesicle size normally ranges between 20—30,000 nm in diameter. The liquid film between lamellae is usually 3—10 nm.

In the broadest terms, liposomes are prepared from one or more surfactants. Though it has been thought that any type of surfactant could be used in liposomes, e.g. cationic, neutral or anionic surfactants, experience with positively charged liposomes has indicated several problems which have not been fully addressed to date. The tertiary amines which have to date been employed in preparing liposomes have either not been sufficiently chemically stable to allow for the storage of the vesicle itself (short shelf life) or the structure of the amines has been such that they can be leached out of the liposome structure. Also one of the tertiary amines, stearoylamine, has toxicity concerns which limit its use as a component of a liposome. Compounds of Formula I do not suffer from these infirmities. The ether linkage of the compounds of formula I is highly stable in liposomes. The compounds of formula I are not leached out of nor do they otherwise migrate out of the liposome matrix as do stearoyl amines and other tertiary amines. Toxicity is not a concern with the compounds of Formula I.

Positively charged pharmaceutical formulations, particularly liposomes, are pharmaceutically advantageous. Mammalian cells are negatively charged so the presentation of positively charged materials results in better attachment to and absorption by membranes.

Typically, the liposome formulations of this invention will be comprised of 0.05 to 10% drug by weight, 1 to 20% surfactant by weight of which a compound of formula I comprises 1 to 100% of this surfactant component and an aqueous solution, that is, water which may or may not contain salts and buffers, in a quantity sufficient to make 100% by volume. Particularly preferred are formulations which contain 1 to 5% drug and a compound of formula I comprises 50% or more by weight of the surfactant component. Most preferred is a formulation which contains 5% drug by weight, a compound of formula I comprises the only surfactant component which component is present in an amount of 20% by weight and an amount of aqueous solution sufficient (q.s.) to make 100% by volume.

Formulations of this invention, particularly liposomes, made with the compounds of formula I will exhibit the properties of a positively charged entity when compounds of formula I, comprising 1% or more by weight of the total weight, are used with a neutral liposome-forming material. Thus, other excipients, surfactants and the like which are used for making liposomes, can be used in these formulations. One may use any combination of secondary surfactants with the compounds of formula I so long as there is 1% or more of a compound of formula I present in the formulation.

Secondary surfactants which can be used are, for example, ternary or complex lipids, glycerides, cerides, etholides and sterides, namely one of several compounds wherein the hydrophilic group is phosphate, carboxylate, sulfate, amino, hydroxyl or choline group; and the lipophilic group is alkyl or alkenyl, polyoxyalkylene or an alkyl group substituted with at least one aromatic or cycloalkyl group. Polyethyleneoxy or glycol groups may be used. Additional surfactants suitable for incorporation into these formulations can be found in the *McCutcheon's Detergents and Emulsifiers* and *McCutcheon's Functional Materials,* Allured Pub. Co., Ridgewood, N.J., U.S.A.

Preferred secondary surfactants are phospholipid-related materials such as, for example, lecithin, phosphatidyl ethanolamine, lysolecithin, lysophosphatidyl-ethanolamine, phosphatidylserine, phosphatidylinositol, sphingomyelin, cephalin, cardiolipin, phosphatidic acid, cerebrosides, dicetyl phosphate, phosphatidylcholine and dipalmitoylphosphatidylcholine. Additional, non-phosphorus-containing lipids are, for instance, cetyl palmitate, glyceryl ricinoleate, hexadecyl stearate, ispropyl myristate, amphoteric acrylic polymers, triethanolamine-lauryl sulfate, alkanoyl-aryl sulfonates, and the like.

Additional additives may be long chain alcohols and diols sterols, for example, cholesterol; phosphoric esters of fatty alcohols, for example, sodium dicetyl phosphate; alkylsulfates, for example, sodium cetyl sulfate; certain polymers such as polypeptides; and proteins.

Typically, liposomes can be divided into three categories based on their overall size and the nature of the lamellar structure. The three classifications, as developed by the New York Academy Sciences Meeting on, "Liposomes and Their Use in Biology and Medicine," of December 1977, are multi-lamellar vesicles (MLV), small uni-lamellar vesicles (SUV) and large uni-lamellar vesicles (LUV).

SUV's range in diameter from approximately 20—50 nm and consist of a single lipid bilayer surrounding an aqueous compartment. While SUV are single compartmental vesicles of fairly uniform size, MLV vary greatly in size up to 10,000 nm, or thereabouts, are multi-compartmental in their structure and contain more than one bilayer. LUV liposomes are so named because of their large diameter which ranges from about 600 nm to 30,000 nm; they contain more than one bilayer.

Liposomes may be prepared by a number of methods not all of which produce the three different types of liposomes. For example, ultrasonic dispersion by means of immersing a metal probe directly into a suspension of MLV's is a common way for preparing SUV's.

Preparing liposomes of the MLV class usually involves dissolving the lipids in an appropriate organic solvent and then drying down the solvent under a gas or air stream. This leaves behind a thin film of dry lipid on the surace of the container. An aqueous solution is then introduced into the container with shaking in order to free lipid material from the sides of the container. This process disperses the lipid, causing it to form into lipid aggregates or liposomes.

Liposomes of the LUV variety may be made by slow hydration of a thin layer of lipid with distilled water or an aqueous solution of some sort.

Alternatively, liposomes may be prepared by lyophilization. This process comprises drying down lipids to a film under a stream of nitrogen. This film is then dissolved in a volatile solvent, frozen, and placed on a lyophilization apparatus to remove the solvent. To prepare a pharmaceutical formulation containing a drug, a solution of the drug is added to the lyophilized lipids, whereupon liposomes are formed.

A variety of methods for preparing various liposome forms have been described in the periodical and patent literature. For specific reviews and information on liposome formulations, reference is made to reviews by Pagano and Weinstein (*Ann. Rev. Biophysic. Bioeng., 7,* 435—68 (1978)) and Szoka and Papahadjopoulos (*Ann. Rev. Biophysic. Bioeng., 9,* 467—508 (1980)) and additionally to a number of patents, for example, U.S. Patent Nos. 4,229,360; 4,224,179; 4,127,344; 4,193,893; 4,217,344; 4,241,046; 4,078,052; and 4,235,871.

The compounds of this invention may be prepared as a racemic mixture of D,L-isomer or as the individual D or L isomer. Because of the availability of D or L starting materials, certain of these compounds are readily prepared as the individual isomer. However, unless the specific isomer is designated, it should be understood that this invention covers both the pure D- or L- isomers as well as the D,L-racemate.

Compounds of formula 1 have one asymmetric site, (marked above as *), and thus can exist as diastereomers. Individual isomers of compounds of formula 1 are named herein using the IUPAC R-S convention, sometimes called the "sequence rule". A description of the R-S convention may be found, for example, in "Introduction to Organic Chemistry" by A. Streitwieser, Jr. and C. Heathcock, (Macmillan Pub. Co., New York, 1976), pages 110—114. Where appropriate, the optical activity of a compound may be indicated by (+), (−), or (±), referring to the direction in which a solution of the compound rotates a plane of polarized light.

Definitions

Alkyl of 6 to 22 carbon atoms refers to a fully saturated alkane straight or branched chain radical having

6 to 22 carbon atoms including hexyl, heptyl, octyl, nonyl, decyl, undecyl, dodecyl, tridecyl, tetradecyl, pentadecyl, hexadecyl, heptadecyl, octadecyl, nonadecyl, eicosyl, heneicosyl, and docosyl.

Alkenyl of 6 to 22 carbon atoms refers to any unsaturated carbon straight or branched chain radical of 6 to 22 carbon atoms which has 1 or more unsaturated bonds in the radical including hexenyl, heptenyl, octenyl, nonenyl, decenyl, undecenyl, dodenyl, tridecenyl, tetradecenyl, pentadecenyl, hexadecenyl, heptadecenyl, octadecenyl, nonadecenyl, eicosenyl, heneicosoenyl, and docosenyl.

Alkyl of 1 to 8 carbon atoms refers to straight or branched chain alkyl radicals such as methyl, ethyl, propyl and butyl, and the like, up to and including an octyl radical and those radicals which are positional isomers of these compounds.

Aryl refers to benzene or naphthalene.

Aralkyl of 7 to 11 carbons refers to a radical having an alkyl group to which is attached a benzene ring such as the benzyl radical, phenethyl, 3-phenylpropyl, or the like.

Drug refers to any therapeutic or propylactic agent other than a food which is used in the prevention, diagnosis, alleviation, treatment, or cure of disease in man or animal.

A pharmaceutically acceptable anion is an anion which itself is non-toxic or otherwise pharmaceutically acceptable and which does not render the compound pharmaceutically unacceptable. Examples of such anions are the halide anions fluoride, chloride, bromide, and iodide. Inorganic anions such as sulfate, sulfite, phosphate, and nitrate may also be used. Organic anions may be derived from simple organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methane sulfonic acid, ethane sulfonic acid, p-toluenesulfonic acid, and the like.

"Z" refers to the cis form of the compound.

The preferred compounds of formula I are those wherein $R^1$ and $R^2$ are the same and are alkyl of $C_{10}$ to $C_{20}$ carbon atoms, or alkenyl of $C_{10}$ to $C_{20}$ carbon atoms; $R^3$, $R^4$, and $R^5$ are methyl or ethyl; n is 1 to 4 and X is a halide ion. Also preferred are those compounds wherein $R^1$ is alkyl or alkenyl of 14 to 22 carbon atoms and $R^2$ is alkyl or alkenyl radical of 6 to 14 carbon atoms.

Most preferred are the following racemic compounds and the optical isomers thereof:
N-(2,3-di-octadecyloxy)-prop-1-yl-N,N,N-trimethylammonium chloride;
N-(2,3-di-(9-(Z)-octadecenyloxy))-prop-1-yl-N,N,N-trimethylammonium chloride;
N-(2,3-di-(4-(Z)-decenyloxy))-prop-1-yl-N,N,N-trimethylammonium chloride;
N-(2,3-di-hexadecyloxy)-prop-1-yl-N,N,N-trimethylammonium chloride;
N-(2,3-di-decyloxy)-prop-1-yl-N,N,N-trimethylammonium chloride;
N-(2-hexadecyloxy-3-decyloxy)-prop-1-yl-N,N,N-trimethylammonium chloride;
N-(2-hexadecyloxy-3-decyloxy)-prop-1-yl-N,N-dimethylamine hydrochloride;
N-(9,10-di-decyloxy)-dec-1-yl-N,N,N-trimethylammonium chloride;
N-(5,6-di-(9-(Z)-octadecenyloxy))-hex-1-yl-N,N,N-trimethylammonium chloride; and
N-(3,4-di-(9-(Z)-octadecenyloxy))-but-1-yl-N,N,N-trimethylammonium chloride.

Compounds of this invention are made by two procedures which depend on the value of n. Where n is 1, the compounds most analogous to glycerol, the compounds of this invention are derived from D-mannitol. The two central hydroxy groups of mannitol are first protected by formation of a ketal, for example by formation of the acetonide. The four remaining hydroxy groups are then converted to ethers using the appropriate long chain fatty acid. This molecule is then chemically split into two units of aldehyde of 3 carbons each, wherein two carbons are substituted with a long chain alkyl or alkenyl group. The aldehyde functionality is then converted to a tertiary amine and then further converted to ether the acid addition salt or a quaternary ammonium compound. This process is exemplified by Reaction Scheme I as follows.

REACTION SCHEME I

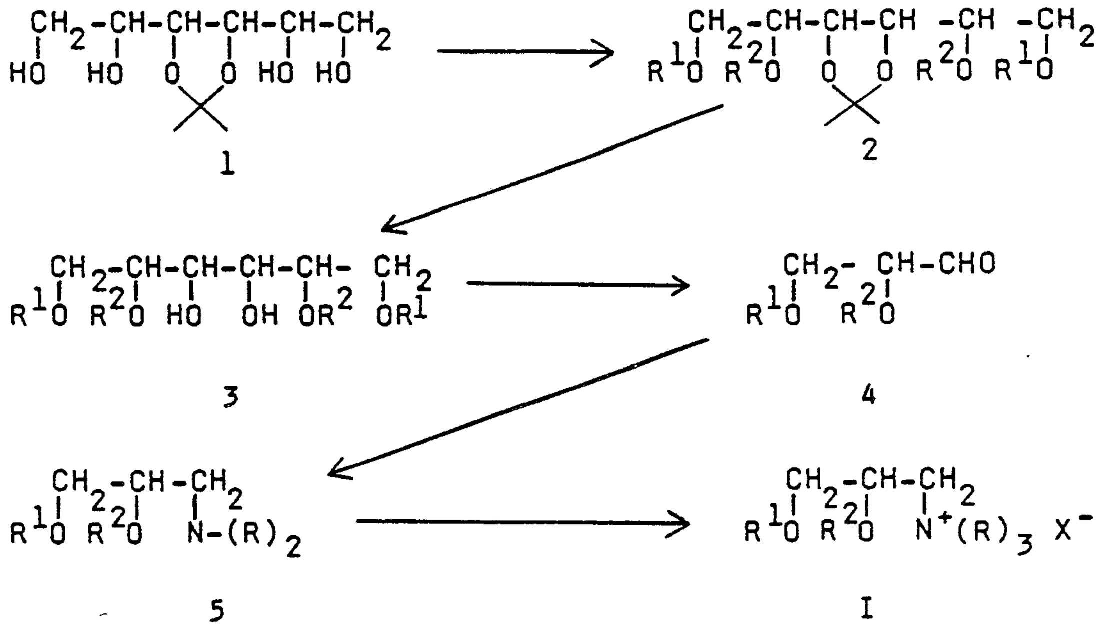

1 2 3 4 5 I

In this reaction scheme $R^1$ and $R^2$ are as defined above and R has the same definition as $R^3$, $R^4$, and $R^5$ above. It should be noted that the term acetonide and the term isopropylidine are used interchangably in the following reaction schemes and examples.

The D-mannitol-3,4-acetonide of formula 1 is a known compound which may be prepared according to the methods of Wiggins, L. F., *J. Chem. Soc.,* 13 (1946); or Morpain, C. & Tisserand, M., *J. Chem. Soc., Perkins Transactions,* 1 (6), 1379 (1979). To form the compound of formula I, perchloric acid is added to a mixture of D-mannitol and 2,2-dimethoxypropane in acetone to form 1,2:3,4:5,6-triisopropylidene-D-mannitol. This compound is then dissolved in 70% acetic acid, heated for 1½ hours, and crystallized from ethyl acetate to give D-mannitol-3,4-acetonide.

To effect formation of compound 2, the acetonide is dissolved in an appropriate polar solvent such as dimethylformamide, diethylformamide, or the like. To this is added a strong base, such as sodium hydride, at room temperature. This mixture is then heated, preferably between about 30° and 100°C, more preferably to about 50°C, with stirring for approximately 30 to 90 minutes, preferably about 60 minutes. To this is then added an alkylating agent of the desired chain length exemplified by the toluenesulfonate ester of oleoyl alcohol or by 1-bromohexadecane. Following addition of the alkylating agent, the temperature is increased to between about 50° and 150°C, preferably about 90°C, with additional stirring over a period of up to 2 hours, preferably about 1 hour. The base/heat/alkylating agent/heat sequence is repeated five times. In the first addition, the base is added in an equal molar amount to the amount of acetonide being used and the alkylating agent is added in an equal molar amount. This sequence of adding a molar amount of base with heating followed by a molar amount of the alkylating ester with heating and stirring is repeated four times (for a total of five times) in order to effect the formation of compound 2.

Compound 3, a mannitol tetraether, is made by hydrolyzing the ketal, illustrated by the acetonide. The hydrolysis is carried out as a single phase reaction using a polar, water soluble organic solvent, such as tetrahydrofuran. Preferably, the hydrolysis will be effected by means of a 10% solution of water in trifluoroacetic acid. The solution of the mannitol tetraether (compound 3) in organic solvent and aqueous acid solution is stirred for approximately one hour at a slightly elevated temperature, approximately 50°C, or thereabouts. The solvent is then evaporated and residual acid removed by azeotropic distillation using a solvent such as toluene.

The aldehyde 4 is made by treating diol 3 with an oxidant, preferably one such as lead tetraacetate, in a solvent best illustrated by chloroform. A slight molar excess of lead tetraacetate is used to effect the reaction. The mixture is stirred at about ambient temperature for up to 4 hours, preferably about 2 hours, at which time the excess lead tetraacetate is quenched by addition of ethylene glycol followed quickly by the addition of a substantial amount of water. The resulting crude aldehyde is recovered by conventional means and may be used without further purification directly in the next step.

To effect the formation of the compound of formula 5, a di-substituted amine hydrochloride (the substituents have the definitions for $R^3$, $R^4$ and $R^5$ above) is dissolved in an alcohol, preferably methanol, to which solution is added a two-thirds molar amount of anhydrous sodium acetate. This mixture is stirred for about an hour at ambient temperature and the resulting sodium chloride is filtered off. The methanol

solution is then added to the crude aldehyde from the preceding paragraph. A second solvent, preferably tetrahydrofuran, is then added to this mixture followed by molecular sieves. To this mixture is then added a reducing agent, preferably sodium cyanoborohydride, in a slight molar excess, and the mixture is stirred at a slightly elevated temperature, preferably about 40 to 60°C, for up to 3 days. This product is then converted to the hydrochloride salt by addition of an organic solvent through which HCl gas has been bubbled.

The quaternary ammonium compound is then prepared by condensing an alkyl or aralkyl chloride into a reaction vessel containing the tri-substituted amine material, after which the reaction vessel is sealed and heated to about 50° to 100°C, preferably about 70°C, for up to 48 hours. This procedure affords the tetrasubstituted ammonium chloride product of formula I (wherein each R can be $R^3$, $R^4$ or $R^5$ as defined above with the proviso that when any of $R^3$, $R^4$, or $R^5$ is defined as aryl then that aryl group is present on the tri-substituted amine).

Alternatively, when compounds of Formula I, wherein n is 1 and $R^1$ and $R^2$ are not the same, are to be made, they can be prepared by the flowchart of Reaction Scheme II which follows.

REACTION SCHEME II

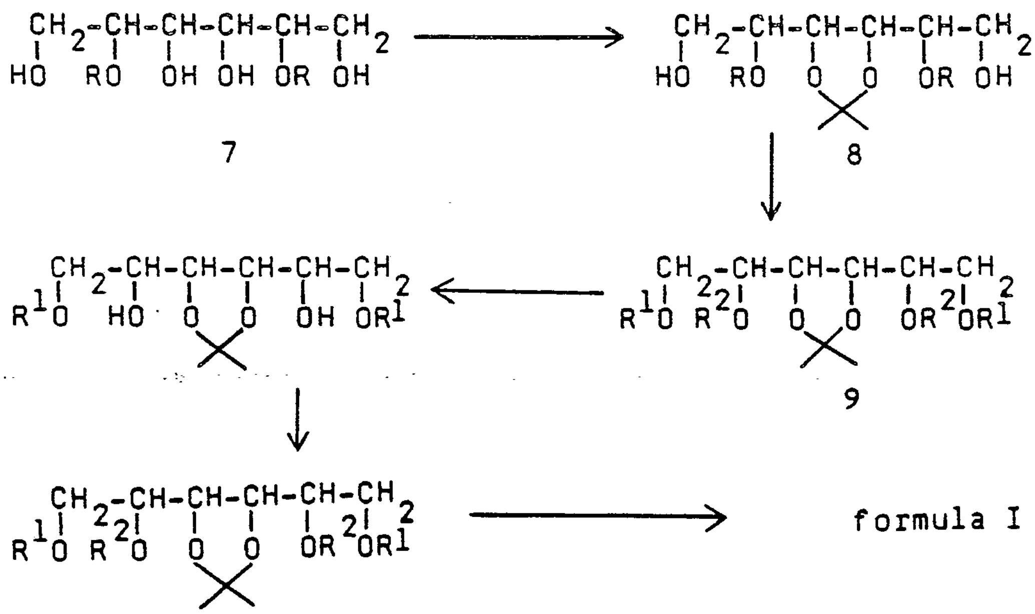
7
8
9
formula I

In this reaction scheme, R is benzyl. $R^1$ and $R^2$ are defined herein above, $R^1$ being different from $R^2$.

The 2,5-dibenzyl-D-mannitol of formula 7 was prepared by the procedure of Baggett, N., & Stribblehil, *J. Chem. Soc. Perkin I,* 1323 (1977). Concentrated sulfuric acid is added to a solution of D-mannitol and benzaldehyde in dimethylformamide. After stirring for 3 days at room temperature, the solution is poured into a mixture of ice water, potassium carbonate, and petroleum ether. The 1,3:4,6-di-O-benzilidine-D-mannitol is then dissolved in benzyl chloride and powdered potassium hydroxide is added. The mixture is heated to 140°C for 3 hours, then cooled and diluted with water. Extraction with chloroform followed by washing with water gives 2,5-di-O-benzyl-1,3:4,6-di-O-benzilidine-D-mannitol. This compound is dissolved in ethanol and water and treated with 1M HCl. After refluxing for 4½ hours the reaction mixture is cooled, quenched with barium carbonate, and evaporated to dryness. The solid residue is triturated with hot ethyl acetate which is then evaporated to yield 2,5-di-O-benzyl-D-mannitol.

The dibenzyl-D-mannitol is dissolved in a dry solvent, for example, acetone, to which is added a half molar amount of copper sulfate and a small amount of concentrated sulfuric acid. This solution is stirred at ambient temperature for about 48 hours, at which time the mixture is quenched by means of a weak base, preferably sodium carbonate, and then stirred for an additional period of time to effect the reaction. The solvent is also the source of the ketal.

The position 1 and position 6 hydroxyl groups are then etherified by means of a strong base and an 1-haloalkyl, or 1-haloalkenyl moiety. The ketal is dissolved in a non-polar solvent, such as xylene, toluene, or the like, to which is added a powdered base, such as powdered potassium hydroxide and the 1-haloalkyl or 1-haloalkenyl material. This mixture is heated at reflux for about 4 hours in order to effect formation of compound 8.

The two benzyl groups are then removed by catalytic hydrogenolysis in an appropriate solvent such as tetrahydrofuran/methanol. A heavy metal catalyst such as 10% palladium on carbon is used. The reaction is carried out in an appropriate hydrogenolysis device, in this instance with heating to about 60° to 80°C, for about 48 hours under about 60 psi of hydrogen.

The diol obtained from the preceding hydrogenolysis is etherified in the same manner described above for preparing compound 9.

Once the tetrasubstituted D-mannitol-3,4-ketal is obtained, it is converted to formula I by the series of steps recited above for conversion of formula 2 to formula I.

Those compounds wherein n is 2 to 8 are prepared form the corresponding triol. The schematic for this reaction sequence is set forth in Reaction Scheme III which follows. This scheme may also be used for preparing compounds where n is 1.

REACTION SCHEME III

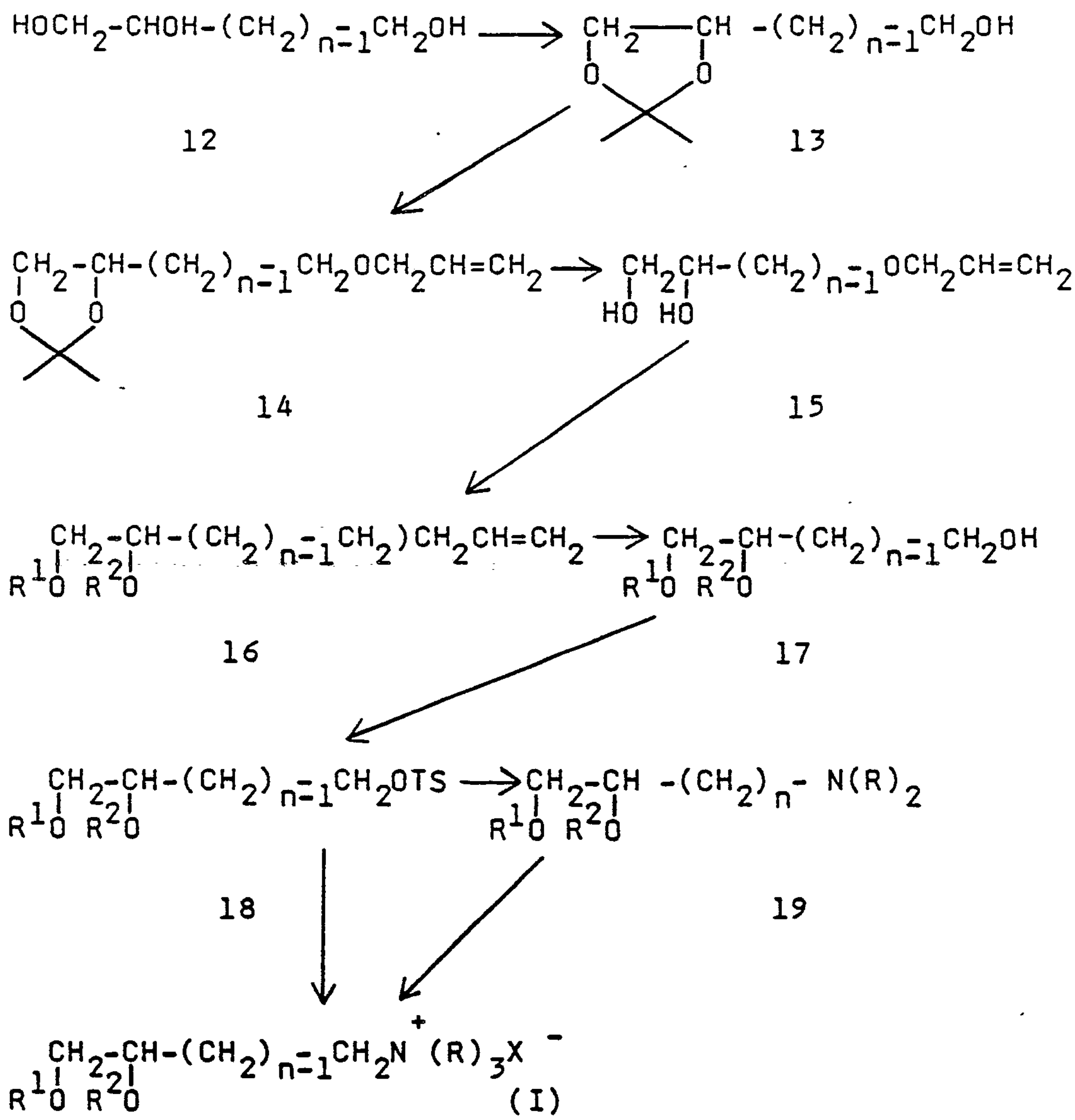

12 13 14 15 16 17 18 19 (I)

In this reaction scheme, $R^1$ and $R^2$ are the same as defined herein above. In formula I, R is the same as $R^3$, $R^4$ and $R^5$.

The compounds of formula 12 are known in the literature or may be purchased from a chemical supply house or may be prepared as indicated herein.

The ketal of formula 13, preferably the acetonide, is prepared by dissolving the appropriate triol in acetone with the addition of a small amount of concentrated sulfuric acid. This reaction may be effected by stirring the solution for up to about 4 hours at room temperature, preferably about 2 hours. The resulting ketal is then recovered by standard separatory means.

The unreacted position-1 hydroxyl group is then protected by forming an allyl ether, compound 14. This reaction is carried out by dissolving the alcohol in a dry dipolar aprotic solvent, such as dimethylformamide. A strong base, such as sodium hydride (an equal molar amount), is added to the alcohol which is stirred at ambient temperature for a set period and then warmed to between 80° and 100°C for an equal period. Allyl chloride, in about a 50% molar excess, is then added at the elevated temperature with stirring.

Stirring and heating is continued for another 30 to 120 minutes, preferably about 60 minutes. The product is then extracted and further purified by chromatographic means.

The ketal is then hydrolyzed to compound 15 by means of a dilute solution of a strong acid, for example 1*N* HCl, the reaction being carried out in a polar solvent, such as methanol, ethanol, or the like. Some heat is added to the reaction mixture to effect the hydrolysis. Preferably, the solution is heated to about 50°C for about 2 hours.

The diol, compound 15, is converted to the diether, compound 16, in the same manner as described above for conversion of formula 1 to formula 2. Here again the etherification is carried out in a dry dipolar aprotic solvent, such as dimethylformamide, using a strong base, such as sodium hydride, and the tosyl ester or halide of the appropriate alkyl or alkenyl alcohol. The reaction is repeated twice using a one molar equivalent of alkylating agent each time. As described previously, the reaction is effected at an elevated temperature, preferably between 50° to 150°C, more specifically at about 90°C.

The position-1 allyl ether, compound 16, is then hydrolyzed by means of Wilkinson's catalyst [tris-(triphenylphosphine), rhodium chloride] in an acid medium. The solvent should be a polar solvent such as ethanol, preferably with a co-solvent such as tetrahydrofuran. The triether/catalyst mixture is refluxed for several hours, preferably about 3 hours, at which time additional acid (1*N* HCl) is added and refluxing continued for several more hours, approximately 3 to 4 hours. These conditions effect hydrolysis of the allyl ether to compound 17.

The alcohol, compound 17, is then converted to the amine by first creating an intermediate p-toluenesulfonate ester, compound 18, to which is added a di-substituted amine to effect formation of the amine compound, compound 19. By way of illustration, the alcohol is dissolved in a suitable solvent, such as pyridine, to which is added p-toluenesulfonyl chloride. This mixture is stirred overnight at ambient temperature, then cooled in ice water and the product recovered by extraction means. The crude product is immediately dissolved in a di-substituted amine, preferably dialkylamine, and placed in a sealed container at ambient temperature for about 1 day to effect formation of the tri-substituted amine.

The alcohol, compound 17, can also be converted to the quaternary amine wherein $R^3$, $R^4$, and/or $R^5$ form a ring with the nitrogen by reacting compound of formula 17 with p-toluenesulfonyl chloride to give the crude tosylate. This compound can be dissolved in methyl ethyl ketone and sodium iodide is added. The mixture is refluxed for 5 hours. The resulting iodopropane compound is dissolved in dichloromethane and the appropriate cyclic nitrogen containing compound is added. The mixture is sealed in a pressure reactor and heated to 100°C for 48 hours followed by chromatography to obtain the appropriate quaternary ammonium compound in which $R^3$, $R^4$, and $R^5$ form a ring with nitrogen, for example, quinuclidine. Alternatively the alcohol, compound 17, is converted to the tosylate, compound 18, which is then reacted in a sealed pressure reactor to form the quaternary compound without going through the formation of the iodine containing compound.

If two of $R^3$, $R^4$, and $R^5$ form a ring with nitrogen, for example, pyrrolidine, piperidine, or morpholine, then alcohol 17 is converted to amine 19 as described above. Amine 19 is then reacted with a di-substituted amine hydrochloride to form the corresponding compound of formula I following the same procedures as given above for the reaction of compound 5 to a compound of formula I.

The tri-substituted amine, compound 19, is most conveniently converted to an acid addition salt, preferably a hydrochloride salt, as a means of isolating the product.

The quaternary ammonium product, formula I, is then prepared in the same manner as described hereinabove for the preparation of formula I.

The following examples are given to illustrate the preparation of the subject compounds and provide examples of their use in pharmaceutical formulations and the preparation of liposomes. These examples are intended to be illustrative only and not to be limiting in any manner.

### Preparation 1

1,2:3,4:5,6-triisopropylidene-D-mannitol

Perchloric acid (3.5 ml, 70%) was added to a mixture of D-mannitol (100 grams) and 2,2-dimethoxypropane (700 ml) in acetone (100 ml). After stirring this mixture at room temperature for 18 hours, sodium bicarbonate (5 grams) was added to the solution. This mixture was stirred at room temperature for 1 hour and then was filtered. The filtrate was concentrated to ½ of the original volume and diluted with water (500 ml) to give the title compound.

### Preparation 2

D-mannitol-3,4-acetonide

1,2:3,4:5,6-triisopropylidene-D-mannitol (90 grams) was dissolved in 70% acetic acid (250 ml) and heated at 45°C for 1.5 hours. The mixture was concentrated *in vacuo* to an oil. This oil was resuspended in toluene (150 ml) and again concentrated *in vacuo*. The resulting oil was dissolved in ethyl acetate (400 ml) and cooled to −5°C. The title compound crystallized from this mixture.

### Example 1

1,3:4,6-Di-O-benzylidene-D-mannitol

Concentrated sulfuric acid (40 ml) was added to a solution of D-mannitol (200 grams) and benzaldehyde (240 ml) in dimethylformamide (600 ml). After stirring this solution at room temperature for 3 days, the mixture was poured into a stirred mixture of ice water (6 liters), potassium carbonate (60 grams) and petroleum ether (1 liter). The resulting solid was collected by filtration, washed with petroleum ether and triturated with hot chloroform to give the title compound, m.p., 190—191°C.

Example 2

2,5-Di-O-benzyl-1,3:4,6-di-O-benzylidene-D-mannitol

Powdered potassium hydroxide (37 grams) was added to 1,3:4,6-di-O-benzylidene-D-mannitol (10 grams) dissolved in benzyl chloride (64 ml). The mixture was heated at 140°C for 3 hours, then cooled and diluted with water (200 ml). Extraction with chloroform, followed by washing with water and evaporation gave a solid, which was crystallized from petroleum ether to give the captioned compound, m.p., 102—103°C.

Example 3

2,5-Di-O-benzyl-D-mannitol

2,5-Di-O-benzyl-1,3:4,6-di-O-benzylidene-D-mannitol (10.9 grams) dissolved in ethanol (150 ml) and water (22 ml) was treated with 1M HCl (7 ml). After refluxing this mixture for 4.5 hours, the reaction was cooled and quenched with barium carbonate, then evaorated to dryness. The solid residue was triturated with hot ethyl acetate, which was then evaporated to give the captioned compound, m.p., 116—117°C.

Example 4

1,2,10-Decanetriol

9-Decen-1-ol (25.0 grams, 160 mM) was dissolved in a solution made up of t-butanol (100 ml), acetone (90 ml) and water (10 ml). To this solution was added trimethylamine-N-oxide (26.6 grams, 240 mM) and 2 ml of a solution of osmium tetroxide (500 mg) in t-butanol (25 ml). The resulting solution was stirred 20 hours under nitrogen then 10% sodium bisulfite was added (50 ml). The mixture was concentrated, then taken up in trichloromethane and washed 2 times with water, dried with $Na_2SO_4$ and concentrated to give 1,2,10-decanetriol as an oil. This material was used without further purification in preparation of the corresponding compound of formula I following the procedures described in Examples 16 through 22 below.

Example 5

2,5-Di-O-benzyl-D-mannitol-3,4-acetonide

2,5-Di-O-benzyl-D-mannitol (48 grams, 133 mM) dissolved in dry acetone (1000 ml) was treated with copper(II)sulfate (10 grams, 62.6 mM) and concentrated sulfuric acid (2 ml). After stirring at room temperature for 48 hours, the mixture was quenched by the addition of solid sodium carbonate, followed by stirring for 3 hours. The reaction mixture was filtered and concentrated and the residue was crystallized from hexane/ethyl acetate to give 38.0 grams of the title compound, m.p. 73—74°C.

The title compound is then reacted using the procedures of examples 16 through 22 below to obtain N-(9,10-di-(9-(Z)-octadecenyloxy))-dec-1-yl-N,N,N-trimethylammonium chloride.

Example 6

2,5-Di-O-benzyl-1,6-didecyl-D-mannitol-3,4-acetonide

A mixture of 2,5-di-O-benzyl-D-mannitol-3,4-acetonide (10.0 grams, 25 mM), powdered potassium hydroxide (23 grams) and decyl bromide (40 ml) in xylene (300 ml) was heated at reflux for 4 hours. The mixture was cooled, diluted with hexanes (300 ml), decanted from excess salts and applied to a column of dry silica gel (1 Kg). Elution with hexanes followed by a gradient of 0 to 50% ether in hexanes gave the title compound as an oil.

Example 7

1,6-Didecyl-D-mannitol-3,4-acetonide

Dibenzyl compound of Example 6 (6.0 grams, 8.8 mM) was dissolved in tetrahydrofuran/methanol (1:1, 100 ml). After bubbling nitrogen through for several minutes, 10% palladium on carbon (1 gram) was added and the mixture was shaken at 70°C under 60 psi hydrogen for 48 hours. The mixture was filtered and concentrated to give the title compound (4.3 grams) as a white solid; m.p. 36—39°C.

Example 8

1,6-Didecyl-2,5-dihexadecyl-D-mannitol-3,4-acetonide

1,6-Didecyl-D-mannitol-3,4-acetonide (4.3 grams, 8.57 mM) and bromohexadecane (7.84 grams, 25.7 mM) were dissolved in xylene (40 ml) and KOH (5.0 grams) was added. This mixture was stirred at reflux for 1.5 hours. After cooling the mixture was decanted onto a column of solica gel (dry, 200 grams), then eluted with hexanes followed by 3% ether in hexanes to give the title compound (7.3 g) as an oil.

The procedures set out in examples 10, 11 and 12 below are followed to form (S) N-(3-decyloxy-2-hexadecyloxy)-prop-1-yl-N,N-dimethylamine hydrochloride, m.p. 45—48°C, $[\alpha]_D^{25}$ −18.7° ($CHCl_3$); and (S) N-(3-decyloxy-2-hexadecyloxy)prop-1-yl-N,N,N-trimethylammonium chloride, m.p. 88—90°C, $[\alpha]_D^{25}$ −24.7° ($CHCl_3$).

Example 9

1,2,5,6-Tetraoleoyl-D-mannitol-3,4-acetonide

D-Mannitol-3,4-acetonide (5.0 grams, 22.52 mM) was dissolved in dimethylformamide (200 ml, distilled from calcium hydride under reduced pressure). To this solution was added sodium hydride (1.08 grams, 22.52 mM, 50% oil dispersion) and the mixture was heated to 50°C and stirred for 1 hour (mechanical stirrer required). To the resulting mixture was aded the toluenesulfonate of oleoyl alcohol (9.5 grams, 22.52 mM). The temperature was increased to 90°C and stirring was continued for 1 hour.

The sequence of addition of sodium hydride (same amount) and stirring 1 hour, then addition of oleoyl tosylate (same amount) and stirring 1 hour, all at a constant 90°C, was repeated 4 more times (total of 5 times). The reaction mixture was allowed to cool to room temperature then poured slowly into a saturated solution of sodium chloride (500 ml). The resulting mixture was extracted with hexanes (3 × 250 ml), dried (potassium carbonate) and concentrated. The crude product was chromatographed over silica gel (1000 grams) eluting with a gradient of from 0 to 5% diethyl ether in hexanes to give 13.93 grams of the title compound as a viscous oil.

Example 10

1,2,5,6-Tetraoleoyl-D-mannitol

To a solution of 1,2,5,6-tetraoleoyl-D-mannitol-3,4-acetonide (24.0 grams, 19.62 mM) in tetrahydrofuran (100 ml) was added $H_2O$:trifluoroacetic acid (1:9, 100 ml). This solution was stirred for 1 hour at 50°C, then concentrated to an oil by rotary evaporation. Toluene (200 ml) was added and evaporated to azeotropically remove the residual acid. The crude material was dissolved in diethyl ether (100 ml) and a saturated solution of ammonium hydroxide in water (10 ml) was added. This mixture was stirred for 2 hours and then the ether phase was washed two times with water, dried (magnesium sulfate) and concentrated. The crude product was suitable for further reaction; a small portion was purified by column chromatography over silica gel (10% ethyl acetate/hexanes) to give an analytical sample of the desired diol as a viscous oil.

Example 11

N-(2,3-Di-(9-(Z)-octadecenyloxy))prop-1-yl-N,N-dimethylamine

The crude diol 1,2,5,6-tetraoleoyl-D-mannitol described in the previous example, was dissolved in chloroform (500 ml) and lead tetraacetate (11.8 grams, 26.0 mM) was added. This mixture was stirred for 2 hours and then ethylene glycol (5 ml) was added followed quickly by water (100 ml). The water phase was drawn off and the organic phase was washed once with saturated sodium chloride solution, dried (magnesium sulfate), and concentrated to an oil to give the crude aldehyde which was used immediately in the next step.

To a solution of dimethylamine hydrochloride (35.5 grams, 435 mM) in methanol (150 ml) was added anhydrous sodium acetate (24 grams, 282 mM). The mixture was stirred for 1 hour and then the resulting sodium chloride was filtered off and the clear methanol solution added to the crude aldehyde. Tetrahydrofuran (150 ml) was added followed by 3 Angström molecular sieves (about 20 grams). Sodium cyanoborohydride (1.5 grams, 23.9 mM) was added and the mixture was stirred at 50°C for three days. The crude reaction mixture was filtered through celite (washing with 1:1 tetrahydrofuran) and the solution was strongly acidified with 1*N* HCl and stirred for ½ hour. The solution was then made strongly basic with 10% NaOH and extracted with diethyl ether (3 × 200 ml). The crude product was purified by column chromatography over silica gel using a gradient of 0 to 10% methanol in chloroform to give the captioned dimethylamino product as a viscous oil.

The hydrochloride salt of the title compound was prepared by dissolving the foregoing product (100 mg) in ether (10 ml) and adding three drops of ethyl acetate saturated with HCl gas. The resulting solution was concentrated and placed under high vacuum for 24 hours. The resulting product was a gummy solid as the S isomer.

In a similar manner, substituting the appropriate diol compound from Example 10, the following compound was prepared:

(S) N-(2,3-di-hexadecyloxy)-prop-1-yl-N,N-dimethylamine hydrochloride, m.p. 49—50°C, $[\alpha]_D^{25}$ −8.39° ($CHCl_3$).

Example 12

N-(2,3-Di-(9-(Z)-octadecenyloxy))-prop-1-yl-N,N,N-trimethylammonium chloride

The dimethyl amino product N-(2,3-di-(9-(Z)-octadecenyloxy))-prop-1-yl-N,N-dimethylamine (10 grams) was placed in a Parr pressure reactor and cooled to −78°C. Methyl chloride (about 50 ml) was condensed into the reaction vessel, which was then sealed and heated to 70°C for 48 hours. The reaction vessel was cooled and opened and the methyl chloride allowed to evaporate under a stream of nitrogen. The crude product was crystallized from acetonitrile to give the title compound as an off-white solid, (S) N-(2,3-di-(9-(Z)-octadecenyloxy)-prop-1-yl-N,N,N-trimethylammonium chloride, $[\alpha]_D^{25}$ −20.0° ($CHCl_3$);

Proceeding in a similar manner, but substituting for N-(2,3-di-(9-(Z)-octadecenyloxy))-prop-1-yl-N,N-dimethylamine the appropriate precursor, the following compounds are prepared:

(S) N-(2,3-di-decyloxy)-prop-1-yl-N,N,N-trimethylammonium chloride, m.p. 87—88°C, $[\alpha]_D^{25}$ −26.5°

($CHCl_3$);

(S) N-(2,3-di-hexadecyloxy)-prop-1-yl-N,N,N-trimethylammonium chloride, $[\alpha]_D^{25}$ −23.4° ($CH_3OH$);

(S) N-(2,3-di-(4-(Z)-decenyloxy))-prop-1-yl-N,N,N-trimethylammonium chloride, wax, $[\alpha]_D^{25}$ 0° ($CHCl_3$);

(S) N-(2,3-di-docosyloxy)-prop-1-yl-N,N,N-trimethylammonium chloride, m.p. 161—163°C, $[\alpha]_D^{25}$ −15.7° ($CHCl_3$); and

(±) N-(2,3-di-(9-(Z)-octadecenyloxy)-prop-1-yl-N,N,N-trimethylammonium chloride, m.p. 35—38°C, NMR (300 MHz, $CDCl_3$) 5.35 (t, J=5Hz, 4H), 4.15—3.90 (m, 2H), 3.80—3.40 (m, 3H), 3.49 (s, 9H), 3.43 (t, J=7Hz, 4H), 2.01 (m, 8H), 1.56 (m, 4H), 1.27 (m, 40H), 0.88 (t, J=7Hz, 6HO.

## Example 13

2,3-Di(9-(Z)-octadecenyl)propan-1-ol

The crude diol 1,2,5,6-tetraoleoyl-D-mannitol described in example 10, was dissolved in chloroform (500 ml) and lead tetraacetate (11.8 grams, 26.0 mM) was added. This mixture was stirred for 2 hours and then ethylene glycol (5 ml) was added followed quickly by water (100 ml). The water phase was drawn off and the organic phase was washed once with saturated sodium chloride solution, dried (magnesium sulfate), and concentrated to an oil to give the crude aldehyde which was used immediately in the next step.

Crude 2,3-di(9-(Z)-octadecenyl)propan-1-al (10.0 grams, 16.9 mM) was dissolved in tetrahydrofuran/methanol (1:1, 200 ml) and cooled to 0°C. Sodium borohydride (3.13 grams, 85.0 mM) was added and the mixture was stirred overnight. The solution was acidified with 1N HCl to pH<2, diluted with ether, washed with water, concentrated and column chromatographed (chloroform) to give the title compound as an oil.

## Example 14

2,3-Di-(9-(Z)-octadecenyloxy)-1-iodopropane

The alcohol 2,3-di(9-(Z)-octadecenyl)propan-1-ol (5.0 grams, 8.36 mM) was dissolved in pyridine (50 ml) and p-toluenesulfonyl chloride (1.91 grams, 10.0 mM) was added. The solution was stirred for 24 hours then poured into ice water, extracted with ether and washed with 1N HCl until the aqueous layer remains acidic. The organic phase was dried (magnesium sulfate) and concentrated to give crude tosylate. The material was dissolved in methyl ethyl ketone (50 ml), sodium iodide (1.5 grams, 10.0 mM) was added and refluxed for 5 hours. The solvent was stripped and the residue was taken up in ether and washed with water. The organic layer was concentrated and chromatographed to give the title compound as an oil.

## Example 15

N-(2,3-Di-(9-(Z)-octadecenyloxy))-prop-1-yl-quinuclidinium chloride

The iodopropane of Example 14 (2.0 grams, 2.82 mM) was dissolved in dichloromethane (1 ml) and quinuclidine (1.57 grams, 14.1 mM) was added. The solution was sealed in a pressure reactor and heated to 100°C for 48 hours. The crude product was chromatographed over a small plug of silica gel (0 to 5% methanol in chloroform) and then ion exchanged over dowex 2-X8 (chloride form, eluting with methanol) to give the title compound, (S) N-(2,3-di-(9-(Z)-octadecenyloxy))-prop-1-yl-quinuclidinium iodide, m.p. 81—83°C, $[\alpha]_D^{25}$ −33.5° ($CHCl_3$);

In a similar manner, but substituting the appropriate starting material, the following compounds were prepared:

(±) N-methyl-N-(2,3-di-hexadecyloxy)-prop-1-yl-pyrrolidinium chloride, m.p. 71—73°C;

(±) N-methyl-N-(2,3-di-hexadecyloxy)-prop-1-yl-piperidinium chloride, m.p. 111—116°C;

(±) N-methyl-N-(2,3-di-hexadecyloxy)-prop-1-yl-morpholinium chloride, m.p. 118—121°C.

## Example 16

5,6-isopropylidine-hexan-1-ol

1,2,6-Hexanetriol (31 grams, 0.23 mM) was stirred with acetone (150 ml). To this mixture was added concentrated sulfuric acid (5 drops). The resulting solution was stirred for 2 hours at room temperature. The reaction solution was diluted with diethyl ether, washed with saturated sodium bicarbonate solution, dried (magnesium sulfate) and concentrated to give the title compound (31 grams) as a clear oil.

## Example 17

5,6-isopropylidene-hexan-1-allyl ether

The hexan-1-ol of Example 16 (30 grams, 172 mM) was dissolved in dry dimethylformamide (500 ml). To this solution was added sodium hydride (8.28 grams, 172 mM, 50% oil dispersion) and the mixture was stirred for ½ hour at room temperature then warmed to 90°C over ½ hour. To this mixture was added allyl chloride (21 ml, 258 mM) and the stirring was continued for 1 hour. After cooling, the mixture was poured into water and extracted with ether (2 × 100 ml). The combined ether extracts were washed with brine, dried (magnesium sulfate) and concentrated. Chromatography over silica gel (10% ether in hexanes) gave the title product as a clear oil; bp = 70°C at 0.01 mmHg.

Example 18

5,6-di-hydroxy-hexan-1-allyl ether

In ethanol (100 ml) was dissolved 5,6-isopropylidene-hexan-1-allyl ether (20 grams, 93.9 mM) to which was added 20 ml of 1*N* HCl. The solution was then heated to 50°C for 2 hours. The resulting solution was concentrated, then taken up in chloroform (100 ml) and washed with brine (2 × 10 ml), dried (sodium sulfate) and concentrated to give the title compound as a clear oil.

Example 19

5,6-Di(9-(Z)-octadecenyloxy)-hexan-1-allyl ether

The product of Example 18 (3.45 grams, 19.83 mM) was dissolved in dry dimethylformamide (60 ml). To this solution was added sodium hydride (951 mg, 19.8 mM). The mixture was heated to 90° and oleoyl tosylate (8.37 grams, 19.8 mM) was added. Stirring was continued for 1 hour at which time a second equivalent of sodium hydride (951 mg, 19.8 mM) was added. After 15 minutes a second equivalent of oleoyl tosylate (8.37 grams, 198 mM) was added and stirring was continued for 1 hour. The reaction mixture was poured into water and extracted with ether (2 × 100 ml). Column chromatography over silica gel (0 to 5% ether in hexanes) gave 3.5 grams of the title compound as a clear oil.

Example 20

5,6-Di(9-(Z)-octadecenyloxy)-hexan-1-ol

The triether of Example 19 (3.20 grams, 4.74 mM) was dissolved in ethanol/tetrahydrofuran (1:1, 30 ml) and Wilkinson's catalyst (tris(triphenylphosphine), rhodium chloride, 200 mg) was added followed by 0.1*N* HCl (1 ml). This mixture was refluxed for 3 hours then 1 *N* HCl (5 ml) was added and refluxed 4 hours. The solution was cooled and concentrated. Diethyl ether was added and washed with brine, dried (magnesium sulfate), concentrated and chromatographed over silica gel (5 to 50% ether in hexanes) to give 2.56 grams of the title alcohol as an oil.

Example 21

(±) N-(5,6-di-(9-(Z)-octadecenyloxy))-hex-1-yl-N,N-dimethyl amine

The substituted hexan-1-ol from Example 20 (2.50 grams, 3.94 mM) was dissolved in pyridine (20 ml) and p-toluenesulfonyl chloride (0.90 grams, 4.73 mM) was added. This mixture was stirred overnight at room temperature then poured into ice water and stirred ½ hour. The resulting mixture was extracted with ether and the ether phase was washed with 0.1*N* HCl, dried (magnesium sulfate) and concentrated. This crude intermediate was immediately dissolved in dimethylamine and placed in a sealed tube at room temperature for 20 hours. The tube was cooled to 0°C and opened. The dimethylamine was allowed to evaporate under a stream of nitrogen. Column chromatography of the crude product over silica gel (0 to 5% methanol in chloroform) gave the title product as a very thick oil. The hydrochloride was prepared as described in Example 11. This was also an oil, NMR (300 MHz, $CDCl_3$) 5.40—5.30 (m, 4H), 3.65—3.50 (m, 1H), 3.50—3.30 (m, 6H), 3.05—2.90 (m, 2H), 2.79 (s, 6H), 2.10—1.65 (m, 11H), 1.65—1.45 (m, 8H), 1.45—1.15 (m, 44H), 0.95—0.80 (m, 6H).

In a similar manner, but substituting the appropriate starting material, the following compounds were prepared:

(±) N-(3,4-di-(9-(Z)-octadecenyloxy))-but-1-yl-N,N-dimethylamine hydrochloride, oil, NMR (90 MHz, $CDCl_3$) 5.33 (t, J=5Hz, 4H), 3.85—3.15 (m, 18H), 2.20—1.80 (m, 8H), 1.70—1.00 (m, 50H), 0.88 (t, J=7Hz, 6H);

(±) N-(9,10-di-(9-(Z)-octadecenyloxy))-dec-1-yl-N,N-dimethylamine hydrochloride, wax, NMR (90 MHz, $CDCl_3$) 5.34 (t, J=5Hz, 4H), 4.65—4.25 (m, 9H), 2.81 (s, 3H), 2.75 (s, 3H), 2.20—1.75 (m, 8H), 1.75—1.00 (m, 62H), 0.88 (t, J=7Hz, 6H).

Example 22

(±) N-(5,6-Di-(9-(Z)-octadecenyloxy))-hex-1-yl-N,N,N-trimethylammonium chloride

This product was prepared in the same manner as set out in Example 12.

(±) N-(5,6-di-(9-(Z)-octadecenyl))-hex-1-yl-N,N-dimethyl-amine hydrochloride, oil, NMR (300 MHz, $CDCl_3$) 5.35 (t, J=5Hz, 4H), 3.70—3.30 (m, 7H), 2.97 (m, 2H), 2.79 (s, 6H), 2.10—1.70 (m, 10H), 1.70—1.10 (m, 59H), 0.88 (t, J=7Hz, 6H);

(±) N-(5,6-di-(9-(Z)-octadecenyloxy))-hex-1-yl-N,N,N-trimethylammonium chloride, oil;

In a similar manner, using the appropriate starting material, the following compounds were prepared:

(±) N-(9,10-di-(9-(Z)-octadecenyloxy))-dec-1-yl-N,N,N-trimethylammonium chloride, oil, NMR (300 MHz, $CDCl_3$) 5.40—5.30 (t, J=tHz, 4H), 3.70—3.30 (m, 9H), 3.46 (s, 9H), 2.10—1.90 (m, 8H), 1.85—1.65 (m, 2H), 1.60—1.20 (m, 50H), 0.88 (t, J=7Hz, 6H);

(±) N-(3,4-di-hexadecyloxy)but-1-yl-N,N,N-trimethylammonium chloride, m.p. 177—179°C.

Example 23

The following compositions illustrate the use of the instant compounds in pharmaceutical formulations.

1) Thirty-four mg of N-(2,3-di-(9-(Z)-octadecenyloxy))-prop-1-yl-N,N,N-trimethylammonium chloride and 6.3 mg of 9-(1,3-dihydroxy-2-propoxymethyl) guanine dipalmitate were dissolved in chloroform/

methanol (2:1: 2 ml). Solvent was removed under a stream of nitrogen and placed *in vacuo* overnight. The dried film was suspended in 1 ml of 50 mM phosphate buffered saline, pH 7.4 and sonicated to clarity.

2) A topical formulation was prepared by dissolving 0.025 mg of fluocinolone acetonide [6α, 9α-difluoro-11β, 16α, 17α, 21-tetrahydroxy-pregna-1,4-diene-3,20-dione 16,17-acetonide] 0.25 grams of N-(2,3-di-(9-(Z)-octadecenyloxy))-prop-1-yl-N,N,N-trimethyl ammonium chloride in 20 ml of dichloromethane. The solvent was evaporated under a stream of nitrogen gas until a dry film was obtained. The film mixture was placed under vacuum overnight to evaporate off the dichloromethane completely. The dry film was then suspended in 25 ml of 1% sodium chloride solution. The suspension was sonicated until visually clear.

3) Fluocinonide [6α, 9α-difluoro-11β, 16α, 17α,21-tetrahydroxy-pregna-1,4-diene-3,20-dione, 16,17-acetonide-21-acetate] 0.025 grams and 1.0 grams of N-(2,3-di-(9-(Z)-octadecenyloxy))-prop-1-yl-N,N,N-trimethyl ammonium chloride were dissolved in 20 ml of dichlormethane which was then evaporated under a stream of nitrogen gas until a dry film is obtained. This film mixture was placed under vacuum overnight to evaporate off residual dichloromethane. The resulting film was suspended in 25 ml of 1% sodium chloride solution and sonicated until visually clear.

4) There was dissolved 160 mg N-(2,3-di-(9-(Z)-octadecenyloxy))-prop-1-yl-N,N,N-trimethylammonium chloride and 20 mg butoconazole nitrate [1-[4-(4-chlorophenyl)-2-(2,6-dichlorophenylthio)-n-butyl]imidazole nitrate] in 2 mls of chloroform. The chloroform was removed under a stream of nitrogen and the residue was placed under vacuum overnight to remove residual chloroform. The resulting film was suspended in 2 mls of purified water by hand shaking and vortexing.

5) Diarachidoylphosphatidyl choline, 60 mg, and 5.4 mg N-(2,3-di-(9-(Z)-octadecenyloxy))-prop-1-yl-N,N,N-trimethylammonium chloride was dissolved in 2 ml of chloroform which was removed under a stream of nitrogen and placed under vacuum overnight to remove residual solvent. The resulting film was suspended in 2 mls of purified water containing 20 million units of beta-interferon by gentle trituration to avoid excessive foaming.

6) Thirty mg of N-(2,3-di-(9-(Z)-octadecenyloxy))-prop-1-yl-N,N,N-trimethylammonium chloride and 3 mg of 6-O-stearoyl-N-acetyl-muramyl-L-α-aminobutyryl-D-isoglutamine were dissolved in chloroform. A nitrogen stream was used to remove the majority of the solvent, the residue being removed under vacuum. The resulting film was suspended in 1 ml of purified water and treated with ultrasound until clear.

7) Distearoylphosphatidyl choline, 2.22 mg, 1 mg N-(2,3-di-(9-(Z)-octadecenyloxy))-prop-1-yl-N,N,N-trimethyl ammonium chloride and 0.23 mg of cholesterol were dissolved in 1 ml chloroform. Solvent was removed under a stream of nitrogen and the residue placed under vacuum overnight. The dried film was suspended in 6 mM phosphate buffered saline containing 8% Triton X-100 (0.5 ml). To this was added 50 µg of lectin affinity column purified bovine herpes antigen. Then 1 ml of packed BioBeads was added (to remove Triton X-100) and shaken gently for 2 hours at 55°C after which the BioBeads were decanted.

8) 14 Micromoles of dioleoylphosphatidyl choline and 6 micromoles of N-(2,3-di-(9-(Z)-octadecenyloxy))-prop-1-yl-N,N,N-trimethylammonium chloride were dissolved in 2 ml of chloroform and then dried down under a stream of nitrogen. The resulting dried film was placed under vacuum for ½ hour, after which the film was then dissolved into 1 ml of cyclohexane, transferred to a 100 ml round-bottomed flask and frozen on dry ice. The flasks were then attached to a lyophilization apparatus and the cyclohexane removed. Murine gamma interferon solution [0.2 ml (500,000 units/ml) was suspended in a buffer containing 10 mM monopotassium phosphate, 2 mM sodium chloride and 3 mM potassium chloride (adjusted to pH 8.0 with potassium hydroxide)] was then added to the lyophilized lipids which caused formation of liposomes. The liposomes were then diluted to a convenient concentration with more phosphate buffer as needed.

In a similar manner other concentrations of drug in liposome can be prepared. By varying the amount of aqueous solution added to the film, the concentration of drug in the final liposome formulation can be varied between 0.05 and 10% by weight.

Example 24

The ability to solubilize a drug is increased by use of the compounds and liposomes of this invention. In this way a greater concentration of a normally insoluble or sparingly soluble drug can be presented to the body.

For example, 1-[4-(4-chlorophenyl)-2-(2,6-dichlorophenylthio)-n-butyl] imidazole nitrate without the presence of any of the compounds of this invention is insoluble in aqueous buffer (phosphate buffered saline, pH 7.4).

A 0.3% soluble preparation of the above drug was prepared using the following method:

18 mg of 1-[4-(4-chlorophenyl)-2-(2,6-dichlorophenylthio)-n-butyl] imidazole nitrate and 482 mg of N-(2,3-di-(9-(Z)-octadecenyloxy)-prop-1-yl-N,N,N-trimethylammonium chloride were dissolved in methylene dichloride. The methylene dichloride was evaporated under a stream of nitrogen and the dried film placed under vacuum overnight. The dried film was suspended in phosphate buffered saline, pH 7.4, and sonicated to clarity.

**Claims for the Contracting States: BE CH DE FR GB IT LI LU NL SE**

1. A compound of the formula

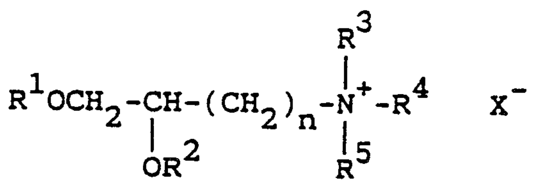

I

or an optical isomer thereof wherein $R^1$ and $R^2$ are the same or different and are an alkyl or alkenyl group of 6 to 22 carbons; $R^3$, $R^4$ and $R^5$ are the same or different and are hydrogen, alkyl of 1 to 8 carbon atoms, aryl, aralkyl of 7 to 11 carbons, or when two or three of $R^3$, $R^4$, and $R^5$ are taken together form quinuclidino, pyrrolidino, piperidino, or morpholino; n is 1 to 8; and X is a pharmaceutically acceptable anion.

2. A compound according to claim 1 wherein $R^1$ and $R^2$ are the same and are alkenyl of 10 to 20 carbon atoms, $R^3$, $R^4$, and $R^5$ are hydrogen, methyl or ethyl and n is 1 to 4.

3. A compound according to claim 2 selected from

(±) N-(2,3-di-(9-(Z)-octadecenyloxy))-prop-1-yl-N,N-trimethylammonium chloride or an optical isomer thereof;

(±) N-(2,3-di-(4-(Z)-decenyloxy))-prop-1-yl-1-(trimethylammonium chloride or an optical isomer thereof;

(±) N-(3,4-di-(9-(Z)-octadecenyloxy))-but-1-yl-N,N,N-trimethylammonium chloride or an optical isomer thereof; and

(±) N-(5,6-di-(9-(Z)-octadecenyloxy))-hex-1-yl-N,N,N-trimethylammonium chloride or an optical isomer thereof.

4. A compound according to claim 1 wherein $R^1$ and $R^2$ are the same and are alkyl of 10 to 20 carbon atoms, $R^3$, $R^4$, and $R^5$ are hydrogen, methyl or ethyl, n is 1 to 4 and X is a halide ion.

5. A compound according to claim 4 selected from

(±) N-(2,3-di-hexadecyloxy)-prop-1-yl-N,N,N-trimethylammonium chloride or an optical isomer thereof; and

(±) N-(2,3-di-octadecyloxy)-prop-1-yl-N,N,N-trimethylammonium chloride.

6. A compound according to claim 1 wherein $R^1$ is alkyl or alkenyl of 14 to 22 carbon atoms; $R^2$ is alkyl or alkenyl of 6 to 14 carbon atoms, $R^3$, $R^4$, and $R^5$ are hydrogen, methyl or ethyl; n is 1 to 4 and X is a halide ion.

7. A compound according to claim 6 selected from

(±) N-(2-hexadecyloxy-3-decyloxy)-prop-1-yl-N,N,N-trimethylammonium chloride or an optical isomer thereof; and

(±) N-(2-hexadecyloxy-3-decyloxy)-prop-1-yl-N,N-dimethylamine hydrochloride.

8. A liposome formulation which comprises 0.05 to 10% by weight of a drug, 1 to 20% surfactant component by weight of which a compound of formula I of any one of the preceding claims comprises 1 to 100% of this surfactant component, and an aqueous solution in a quantity sufficient to make 100% by volume.

9. A pharmaceutical formulation which comprises 0.05 to 10% by weight of a drug, 1 to 20% surfactant component by weight of which a compound of formula I of any one of claims 1 to 7 comprises 1 to 100% of this surfactant component, and an aqueous solution in a quantity sufficient to make 100% by volume.

10. The formulation of claim 9 wherein said drug is 9-(1,3-dihydroxy-2-propoxymethyl)quanine dipalmitate, 6α,9α-difluoro-11β,16α,17α21-tetrahydroxy-pregna-1,4-diene-3,20-dione-16,17-acetonide (fluocinolone acetonide), 6α,9α-difluoro-11β,16α,17α,21-tetrahydroxy-pregna-1,4-diene-3,20-dione-16,17-acetonide-21-acetate (fluocinonide), 1-[4-(4-chlorophenyl)-2-(2,6-dichlorophenylthio)-n-butyl] imidazole nitrate (butaconazole nitrate), beta-interferon, gamma-interferon, 6-O-stearoyl-N-acetylmuramyl-L-α-aminobutyryl-D-isoglutamine or a herpes antigen.

11. A formulation according to claim 9 or claim 10 wherein said drug comprises 1 to 5% and formula I comprises 50% or more of the surfactant component.

12. A formulation according to claim 9, 10 or 11 having a compound of formula I wherein $R^1$ and $R^2$ are the same and are alkyl or alkenyl of 10 to 20 carbon atoms, $R^3$, $R^4$ and $R^5$ are hydrogen, methyl or ethyl, n is 1 to 4 and X is a halide ion.

13. A formulation according to claim 12 having a compound of formula I which is selected from

(±) N-(2,3-di-hexadecyloxy)-prop-1-yl-N,N,N-trimethylammonium chloride or an optical isomer thereof;

(±) N-(2,3-di-octadecyloxy)-prop-1-yl-N,N,N-trimethylammonium chloride or an optical isomer thereof; and

(±) N-(2,3-di-(9-(Z)-octadecenyloxy))-prop-1-yl-N,N,N-trimethylammonium chloride or an optical isomer thereof.

14. A formulation according to claim 9, 10 or 11 having a compound of formula I wherein $R^1$ and $R^2$ are the same and are alkyl of 10 to 20 carbon atoms, or alkenyl of 10 to 20 carbon atoms and $R^3$, $R^4$, and $R^5$ are hydrogen, methyl or ethyl and n is 1 to 4.

15. A formulation according to claim 9, 10 or 11, wherein $R^1$ is alkyl or alkenyl of 14 to 22 carbon atoms and $R^2$ is a different alkyl group of 6 to 14 carbon atoms; $R^3$, $R^4$, and $R^5$ are hydrogen, methyl or ethyl; n is 1 to 4; and X is a halide ion.

16. A process for the preparation of a compound of any one of claims 1 to 7 which comprises reacting a compound of the formula

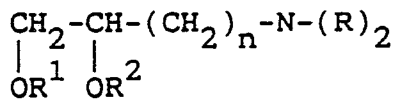

wherein $R^1$, $R^2$ and n are as defined above and each R group is defined as $R^3$, $R^4$, and $R^5$ are defined above, with an alkyl or aralkyl chloride to form a compound of formula I.

17. A process for preparation of a compound of the formula

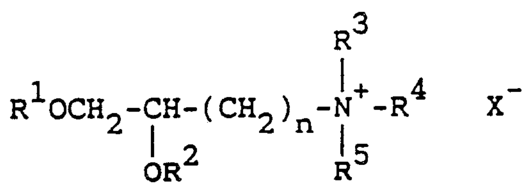

I

or an optical isomer thereof wherein $R^1$ and $R^2$ are the same or different and are an alkyl or alkenyl group of 6 to 22 carbons; none or one of $R^3$, $R^4$ and $R^5$ is hydrogen, alkyl of 1 to 8 carbon atoms, aryl, or aralkyl of 7 to 11 carbons and two or three of $R^3$, $R^4$, and $R^5$ are taken together to form quinuclidino, pyrrolidino, piperidino, or morpholino; n is 1 to 8; and X is a pharmaceutically acceptable anion, which comprises

(a) reacting a compound of the formula

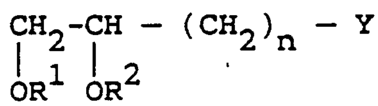

wherein $R^1$, $R^2$ and n are as defined above and Y is a halide or p-toluene sulfonyl group with the appropriate hydrocarbon selected from quinuclidine, pyrrolidine, piperidine, or morpholine, optionally followed by

(b) reacting the product of step (a) wherein two of $R^3$, $R^4$ and $R^5$ are taken together to form pyrrolidino, piperidino, or morpholino with an alkyl or aralkyl chloride to form a compound of formula I.

18. A compound of the formula

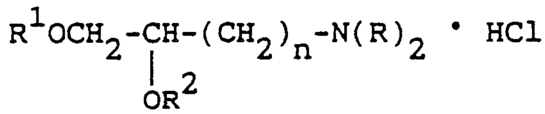

or an optical isomer thereof wherein $R^1$ and $R^2$ are the same or different and are an alkyl or alkenyl group of 6 to 22 carbons; each R is the same or different and are hydrogen, alkyl of 1 to 8 carbon atoms, aryl, or aralkyl of 7 to 11 carbons or two R groups are taken together to form pyrrolidino, piperidino, or morpholino; and n is 1—8.

19. A process for the preparation of a compound of formula I wherein n is 1 which process includes reductive amination of a compound of formula III

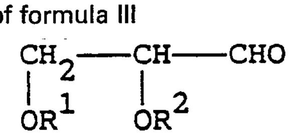

III

wherein $R^1$ and $R^2$ are as defined above, to form a compound of formula

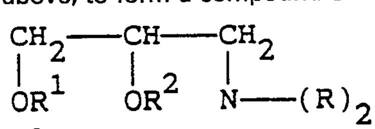

wherein R is defined as $R^3$, $R^4$ and $R^5$ as defined above.

20. A process according to claim 19 wherein the compound of formula III is produced from a D-mannitol derivative of formula IV

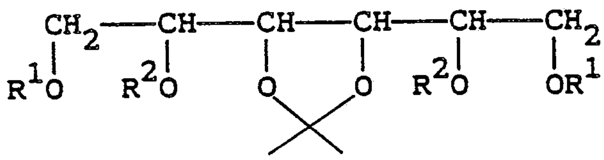

IV

**Claims for the Contracting State: AT**

1. A process for the preparation of a compound of the formula

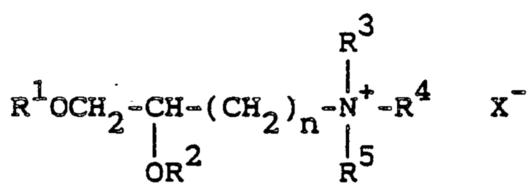

I

or an optical isomer thereof wherein $R^1$ and $R^2$ are the same or different and are an alkyl or alkenyl group of 6 to 22 carbons; $R^3$, $R^4$ and $R^5$ are the same or different and are hydrogen, alkyl of 1 to 8 carbon atoms, aryl, aralkyl of 7 to 11 carbons or when two or three of $R^3$, $R^4$, and $R^5$ are taken together form quinuclidino, pyrrolidino, piperidino, or morpholino; n is 1 to 8; and X is a pharmaceutically acceptable anion, which comprises reacting a compound of the formula

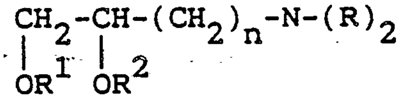

wherein $R^1$, $R^2$ and n are as defined above and each R group is defined as $R^3$, $R^4$, and $R^5$ are defined above, with an alkyl or aralkyl chloride to form a compound of formula I.

2. A process for preparation of a compound of the formula

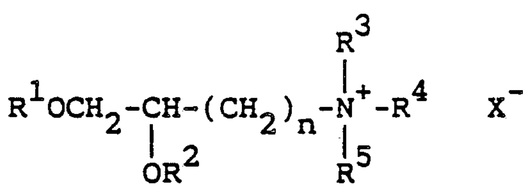

I

or an optical isomer thereof wherein $R^1$ and $R^2$ are the same or different and are an alkyl or alkenyl group of 6 to 22 carbons; none or one of $R^3$, $R^4$ and $R^5$ is hydrogen, alkyl of 1 to 8 carbon atoms, aryl, or aralkyl of 7 to 11 carbons and two or three of $R^3$, $R^4$, and $R^5$ are taken together to form quinuclidino, pyrrolidino, piperidino, or morpholino; n is 1 to 8; and X is a pharmaceutically acceptable anion, which comprises

(a) reacting a compound of the formula

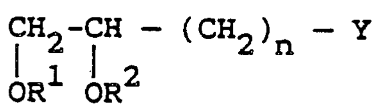

wherein $R^1$, $R^2$ and n are as defined above and Y is a halide or p-toluene sulfonyl group with the appropriate hydrocarbon selected from quinuclidine, pyrrolidine, piperidine, or morpholine, optionally followed by

(b) reacting the product of step (a) wherein two of $R^3$, $R^4$, and $R^5$ are taken together to form pyrrolidino, piperidino, or morpholino with an alkyl or aralkyl chloride to form a compound of formula I.

3. A process for the preparation of a compound of the formula

$$R^1OCH_2-\underset{\displaystyle OR^2}{\underset{|}{CH}}-(CH_2)_n-N(R)_2 \cdot HCl$$

or an optical isomer thereof wherein $R^1$ and $R^2$ are the same or different and are an alkyl or alkenyl group of 6 to 22 carbons; each R is the same or different and are hydrogen, alkyl of 1 to 8 carbon atoms, aryl, or aralkyl of 7 to 11 carbons or two R groups are taken together to form pyrrolidino, piperidino, or morpholino; and n is 1 to 8, which comprises reacting the free base of the compound defined hereinabove with hydrochloric acid.

4. A process according to claim 1 or claim 3 wherein $R^1$ and $R^2$ are the same and are alkenyl of 10 to 20 carbon atoms, $R^3$, $R^4$, and $R^5$ are hydrogen, methyl or ethyl and n is 1 to 4.

5. A process according to claim 4 wherein a compound selected from

(±) N-(2,3-di-(9-(Z)-octadecenyloxy))-prop-1-yl-N,N-trimethylammonium chloride or an optical isomer thereof;

(±) N-(2,3-di-(4-(Z)-decenyloxy))-prop-1-yl-1-(trimethylammonium chloride or an optical isomer thereof;

(±) N-(3,4-di-(9-(Z)-octadecenyloxy))-but-1-yl-N,N,N-trimethylammonium chloride or an optical isomer thereof; and

(±) N-(5,6-di-(9-(Z)-octadecenyloxy))-hex-1-yl-N,N,N-trimethylammonium chloride or an optical isomer thereof, is prepared.

6. A process according to claim 1 or claim 3 wherein $R^1$ and $R^2$ are the same and are alkyl of 10 to 20 carbon atoms, $R^3$, $R^4$, and $R^5$ are hydrogen, methyl or ethyl, n is 1 to 4 and X is a halide ion.

7. A process according to claim 6 wherein a compound selected from

(±) N-(2,3-di-hexadecyloxy)-prop-1-yl-N,N,N-trimethylammonium chloride or an optical isomer thereof; and

(±) N-(2,3-di-octadecyloxy)-prop-1-yl-N,N,N-trimethylammonium chloride is prepared.

8. A process according to claim 1 or claim 3 wherein $R^1$ is alkyl or alkenyl of 14 to 22 carbon atoms; $R^2$ is alkyl or alkenyl of 6 to 14 carbon atoms, $R^3$, $R^4$, and $R^5$ are hydrogen, methyl or ethyl; n is 1 to 4 and X is a halide ion.

9. A process according to claim 8 wherein a compound selected from

(±) N-(2-hexadecyloxy-3-decyloxy)-prop-1-yl-N,N,N-trimethylammonium chloride or an optical isomer thereof; and

(±) N-(2-hexadecyloxy-3-decyloxy)-prop-1-yl-N,N-dimethylamine hydrochloride is prepared.

10. Use of a compound of formula I as defined in any one of the preceding claims in the manufacture of a liposome formulation which comprises 0.05 to 10% by weight of a drug, 1 to 20% surfactant component by weight of which a compound of formula I of any one of the preceding claims comprises 1 to 100% of this surfactant component, and an aqueous solution in a quantity sufficient to make 100% by volume.

11. Use of a compound of formula I as defined in any one of claims 1 to 9 in the manufacture of a pharmaceutical formulation which comprises 0.05 to 10% by weight of a drug, 1 to 20% surfactant component by weight of which a compound of formula I of any one of claims 1 to 7 comprises 1 to 100% of this surfactant component, and an aqueous solution in a quantity sufficient to make 100% by volume.

12. A process according to claim 11 wherein said drug is 9-(1,3-dihydroxy-2-propoxymethyl)quanine dipalmitate, 6α,9α-difluoro-11β,16α,17α,21-tetrahydroxy-pregna-1,4-diene-3,20-dione-16,17-acetonide (fluocinolone acetonide), 6α,9α-difluoro-11β,16α,17α,21-tetrahydroxy-pregna-1,4-diene-3,20-dione-16,17-acetonide-21-acetate (fluocinonide), 1-[4-(4-chlorophenyl)-2-(2,6-dichlorophenylthio)-n-butyl] imidazole nitrate (butaconazole nitrate), beta-interferon, gamma-interferon, 6-O-stearoyl-N-acetylmuramyl-L-α-aminobutyryl-D-isoglutamine or a herpes antigen.

13. A process according to claim 11 or claim 12 wherein said drug comprises 1 to 5% and formula I comprises 50% or more of the surfactant component.

14. A formulation according to claim 11, 12 or 13 wherein in the compound of formula I, $R^1$ and $R^2$ are the same and are alkyl or alkenyl of 10 to 20 carbon atoms, $R^3$, $R^4$ and $R^5$ are hydrogen, methyl or ethyl, n is 1 to 4 and X is a halide ion.

15. A process according to claim 14 wherein the compound of formula I is selected from

(±) N-(2,3-di-hexadecyloxy)-prop-1-yl-N,N,N-trimethylammonium chloride or an optical isomer thereof;

(±) N-(2,3-di-octadecyloxy)-prop-1-yl-N,N,N-trimethylammonium chloride or an optical isomer thereof; and

(±) N-(2,3-di-(9-(Z)-octadecenyloxy))-prop-1-yl-N,N,N-trimethylammonium chloride or an optical isomer thereof.

16. A process according to claim 11, 12 or 13 wherein in the compound of formula I $R^1$ and $R^2$ are the same and are alkyl of 10 to 20 carbon atoms, or alkenyl of 10 to 20 carbon atoms and $R^3$, $R^4$, and $R^5$ are hydrogen, methyl or ethyl and n is 1 to 4.

17. A process according to claim 11, 12 or 13 wherein in the compound of formula I $R^1$ is alkyl or alkenyl of 14 to 22 carbon atoms and $R^2$ is a different alkyl group of 6 to 14 carbon atoms; $R^3$, $R^4$, and $R^5$ are hydrogen, methyl or ethyl; n is 1 to 4; and X is a halide ion.

18. A process for the preparation of a compound of formula I wherein n is 1 which process includes reductive amination of a compound of formula III

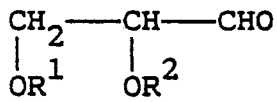

III

wherein $R^1$ and $R^2$ are as defined above, to form a compound of formula

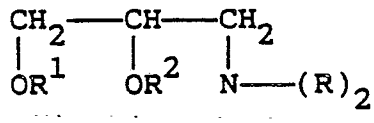

wherein R is defined as $R^3$, $R^4$ and $R^5$ as defined above.

19. A process according to claim 18 wherein the compound of formula III is produced from a D-mannitol derivative of formula IV

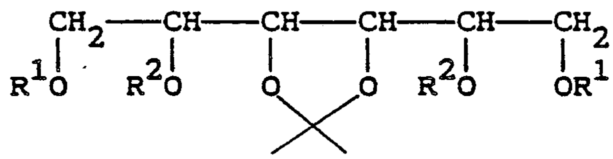

IV

**Patentansprüche für die Vertragsstaaten: BE CH DE FR GB IT LI LU NL SE**

1. Verbindung der Formel

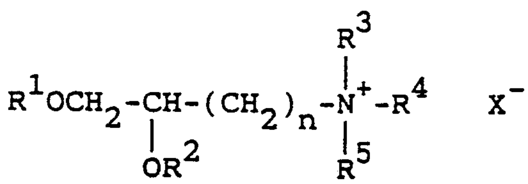

I

oder ein optisches Isomer davon, worin $R^1$ und $R^2$ gleich oder verschieden sind und eine Alkyl- oder Alkenylgruppe mit 6 bis 22 Kohlenstoffatomen sind; $R^3$, $R^4$ und $R^5$ gleich oder verschieden sind und Wasserstoff, Alkyl mit 1 bis 8 Kohlenstoffatomen, Aryl, Aralkyl mit 7 bis 11 Kohlenstoffen sind oder, wenn zwei oder drei der $R^3$, $R^4$ und $R^5$ zusammengefaßt werden, Chinuclidino, Pyrolidino, Piperidino oder Morpholino bilden; n 1 bis 8 ist; und X ein pharmazeutisch annehmbares Anion ist.

2. Verbindung nach Anspruch 1, worin $R^1$ und $R^2$ gleich sind und Alkenyl mit 10 bis 20 Kohlenstoffatomen sind, $R^3$, $R^4$ und $R^5$ Wasserstoff, Methyl oder Ethyl sind und n 1 bis 4 ist.

3. Verbindung nach Anspruch 2, ausgewählt aus

(±) N-(2,3-Di-(9-(Z)-octadecenyloxy))-prop-1-yl-N,N-trimethylammoniumchlorid oder einem optischen Isomer davon;

(±) N-(2,3-Di-(4-(Z)-decenyloxy))-prop-1-yl-1-trimethylammoniumchlorid oder einem optischen Isomer davon;

(±) N-(3,4-Di-(9-(Z)-octadecenyloxy))-but-1-yl-N,N,N-1-trimethylammoniumchlorid oder einem optischen Isomer davon; und

(±) N-(5,6-Di-(9-(Z)-octadecenyloxy))-hex-1-yl-N,N,N-trimethylammoniumchlorid oder einem optischen Isomer davon.

4. Verbindung nach Anspruch 1, worin $R^1$ und $R^2$ gleich sind und Alkyl mit 10 bis 20 Kohlenstoffatomen sind, $R^3$, $R^4$ und $R^5$ Wasserstoff, Methyl oder Ethyl sind, n 1 bis 4 ist und X ein Halogenidion ist.

5. Verbindung nach Anspruch 4, ausgewählt aus

(±) N-(2,3-Di-hexadecyloxy)prop-1-yl-N,N,N-trimethylammoniumchlorid oder einem optischen Isomer davon; und

(±) N-(2,3-Di-octadecyloxy)prop-1-yl-N,N,N-trimethylammoniumchlorid.

6. Verbindung nach Anspruch 1, worin $R^1$ Alkyl oder Alkenyl mit 14 bis 22 Kohlenstoffatomen ist; $R^2$ Alkyl oder Alkenyl mit 6 bis 14 Kohlenstoffatomen ist, $R^3$, $R^4$ und $R^5$ Wasserstoff, Methyl oder Ethyl sind; n 1

bis 4 ist und X ein Halogenidion ist.

7. Verbindung nach Anspruch 6, ausgewählt aus

(±) N-(2-Hexadecyloxy-3-decyloxy)-prop-1-yl-N,N,N-trimethylammoniumchlorid oder einem optischen Isomer davon; und

(±) N-(2-Hexadecyloxy-3-decyloxy)-prop-1-yl-N,N-dimethylaminhydrochlorid.

8. Liposom-Formulierung, umfassend 0,05 bis 10 Gewichtsprozent eines Arzneistoffes, 1 bis 20 Gewichtsprozent einer oberflächenaktiven Komponente, wobei von diesem Gewicht eine Verbindung der Formel I oder irgendeines der vorangehenden Ansprüche 1 bis 100% dieser oberflächenaktiven Komponente umfaßt, und eine wäßrige Lösung in einer Menge, die genügt, 100 Volumenprozent auszumachen.

9. Pharmazeutische Formulierung, umfassend 0,05 bis 10 Gewichtsprozent eines Arzneistoffes, 1 bis 20 Gewichtsprozent einer oberflächenaktiven Komponente, wobei von diesem Gewicht eine Verbindung der Formel I oder irgendeines der Ansprüche 1 bis 7 1 bis 100% dieser oberflächenaktiven Komponente umfaßt, und eine wäßrige Lösung in einer Menge, die genügt, um 100 Volumenprozent auszumachen.

10. Formulierung nach Anspruch 9, worin der Arzneistoff 9-(1,3-Dihydroxy-2-propoxymethyl)guanin-dipalmitat, 6α,9α-Difluor-11β,16α,17α,21-tetrahydroxy-pregna-1,4-dien-3,20-dion-16,17-acetonid (Fluocinolonacetonid), 6α,9α-Difluoro-11β,16α,17α,21-tetrahydroxy-pregna-1,4-dien-3,20-dion-16,17-acetonid-21-acetat (Fluocinonid), 1-[4-(4-Chlorphenyl)-2-(2,6-dichlorophenylthio)-n-butyl]imidazolnitrat (Butaconazolnitrat), beta-Interferon, gamma-Interferon, 6-O-Stearoyl-N-acetylmuramyl-L-α-aminobutyryl-D-isoglutamin oder ein Herpesantigen ist.

11. Formulierung nach Anspruch 9 oder Anspruch 10, worin der Arzneistoff 1 bis 5% umfaßt und Formel I 50% oder mehr der oberflächenaktiven Verbindung umfaßt.

12. Formulierung nach Anspruch 9, 10 oder 11, die eine Verbindung der Formel I aufweist, worin $R^1$ und $R^2$ gleich sind und Alkyl oder Alkenyl mit 10 bis 20 Kohlenstoffatomen sind, $R^3$, $R^4$ und $R^5$ Wasserstoff, Methyl oder Ethyl sind, n 1 bis 4 ist und X ein Halogenidion ist.

13. Formulierung nach Anspruch 12, die eine Verbindung der Formel I aufweist, die ausgewählt ist aus

(±) N-(2,3-Di-hexadecyloxy)-prop-1-yl-N,N,N-trimethylammoniumchlorid oder einem optischen Isomer davon;

(±) N-(2,3-Di-octadecyloxy)-prop-1-yl-N,N,N-trimethylammoniumchlorid oder einem optischen Isomer davon; und

(±) N-(2,3-Di-(9-(Z)-octadecenyloxy))-prop-1-yl-N,N,N-trimethylammoniumchlorid oder einem optischen Isomer davon.

14. Formulierung nach Anspruch 9, 10 oder 11, die eine Verbindung der Formel I aufweist, worin $R^1$ und $R^2$ gleich sind und Alkyl mit 10 bis 20 Kohlenstoffatomen oder Alkenyl mit 10 bis 20 Kohlenstoffatomen sind und $R^3$, $R^4$ und $R^5$ Wasserstoff, Methyl oder Ethyl sind und n 1 bis 4 ist.

15. Formulierung nach Anspruch 9, 10 oder 11, worin $R^1$ Alkyl oder Alkenyl mit 14 bis 22 Kohlenstoffatomen ist und $R^2$ eine verschiedene Alkylgruppe mit 6 bis 14 Kohlenstoffatomen ist; $R^3$, $R^4$ und $R^5$ Wasserstoff, Methyl oder Ethyl sind; n 1 bis 4 ist; und X ein Halogenidion ist.

16. Verfahren zur Herstellung einer Verbindung nach irgendeinem der Ansprüche 1 bis 7, umfassend das Umsetzen einer Verbindung der Formel

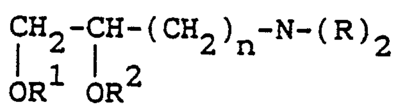

worin $R^1$, $R^2$ und n wie oben definiert sind und jede Gruppe R definiert ist, wie $R^3$, $R^4$ und $R^5$ oben definiert sind, mit einem Alkyl- oder Aralkylchlorid, um ein Verbindung der Formel I zu bilden.

17. Verfahren zur Herstellung einer Verbindung der Formel

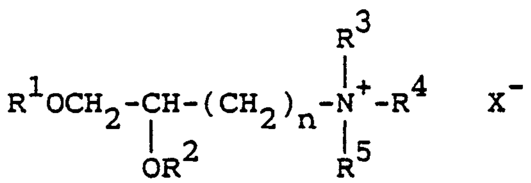

I

oder eines optischen Isomeren davon, worin $R^1$ und $R^2$ gleich oder verschieden sind und eine Alkyl- oder Alkenylgruppe mit 6 bis 22 Kohlenstoffen sind; keines oder eines der $R^3$, $R^4$ und $R^5$ Wasserstoff, Alkyl mit 1 bis 8 Kohlenstoffatomen, Aryl oder Aralkyl mit 7 bis 11 Kohlenstoffen sind, und zwei oder drei der $R^3$, $R^4$ und $R^5$ zusammengefaßt werden, um Chinuclidino, Pyrrolidino, Piperidino oder Morpholino bilden; n 1 bis 8 ist; und X ein pharmazeutisch annehmbares Anion ist, umfassend

(a) das Umsetzen einer Verbindung der Formel

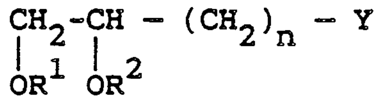

worin $R^1$, $R^2$ und n wie oben definiert sind und Y eine Halogenid- oder p-Toluolsulfonylgruppe ist, mit dem geeigneten Kohlenwasserstoff, ausgewählt aus Chinuclidin, Pyrrolidin, Piperidin oder Morpholin, gegebenenfalls gefolgt von

(b) dem Umsetzen des Produkts von Stufe (a), worin zwei der $R^3$, $R^4$ und $R^5$ zusammengefaßt werden, um Pyrrolidino, Piperidino oder Morpholino zu bilden, mit einem Alkyl- oder Aralkylchlorid um ein Verbindung der Formel I zu bilden.

18. Verbindung der Formel

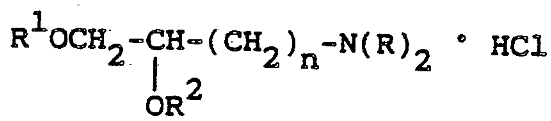

oder ein optisches Isomer davon, worin $R^1$ und $R^2$ gleich oder verschieden sind und eine Alkyl- oder Alkenylgruppe mit 6 bis 22 Kohlenstoffen sind; jedes R gleich oder verschieden ist und Wasserstoff, Alkyl mit 1 bis 8 Kohlenstoffatomen, Aryl oder Aralkyl mit 7 bis 11 Kohlenstoffen ist oder zwei Gruppen R zusammengefaßt werden, um Pyrrolidino, Piperidino oder Morpholino zu bilden; und n 1—8 ist.

19. Verfahren für die Herstellung einer Verbindung der Formel I, worin n 1 ist, einschließend die reduktive Aminierung einer Verbindung der Formel III

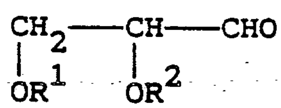

III

worin $R^1$ und $R^2$ wie oben definiert sind, um eine Verbindung der Formel

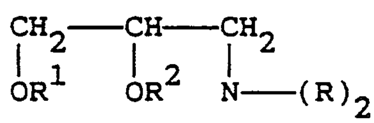

zu bilden, worin R definiert ist, wie $R^3$, $R^4$ und $R^5$ oben definiert sind.

20. Verfahren nach Anspruch 19, worin die Verbindung der Formel III aus einem D-Mannitolderivat der Formel IV

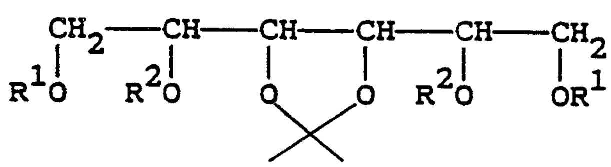

IV

hergestellt wird.

**Patentansprüche für den Vertragsstaat: AT**

1. Verfahren zur Herstellung einer Verbindung der Formel

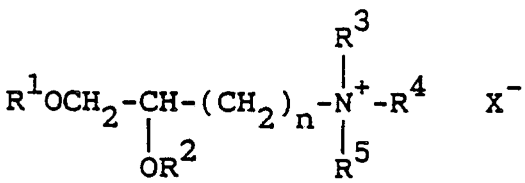

I

oder eines optischen Isomeren davon, worin $R^1$ und $R^2$ gleich oder verschieden sind und eine Alkyl- oder

Alkenylgruppe mit 6 bis 22 Kohlenstoffatomen sind; $R^3$, $R^4$ und $R^5$ gleich oder verschieden sind und Wasserstoff, Alkyl mit 1 bis 8 Kohlenstoffatomen, Aryl, Aralkyl mit 7 bis 11 Kohlenstoffen sind oder, wenn zwei oder drei der $R^3$, $R^4$ und $R^5$ zusammengefaßt werden, Chinuclidino, Pyrrolidino, Piperidino oder Morpholino bilden; n 1 bis 8 ist; und X ein pharmazeutisch annehmbares Anion ist, umfassend das Umsetzen einer Verbindung der Formel

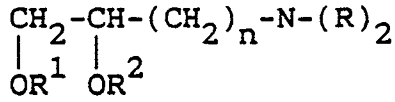

worin $R^1$, $R^2$ und n wie oben definiert sind und jede Gruppe R definiert ist, wie $R^3$, $R^4$ und $R^5$ oben definiert sind, mit einem Alkyl- oder Aralkylchlorid, um ein Verbindung der Formel I zu bilden.

2. Verfahren zur Herstellung einer Verbindung der Formel

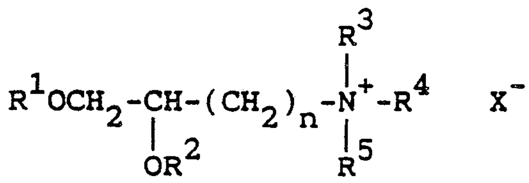

I

oder eines optischen Isomeren davon, worin $R^1$ und $R^2$ gleich oder verschieden sind und eine Alkyl- oder Alkenylgruppe mit 6 bis 22 Kohlenstoffatomen sind; keines oder eines der $R^3$, $R^4$ und $R^5$ Wasserstoff, Alkyl mit 1 bis 8 Kohlenstoffatomen, Aryl oder Aralkyl mit 7 bis 11 Kohlenstoffen sind oder zwei oder drei der $R^3$, $R^4$ und $R^5$ zusammengefaßt werden, um Chinuclidino, Pyrrolidino, Piperidino oder Morpholino zu bilden; n 1 bis 8 ist; und X ein pharmazeutisch annehmbares Anion ist, umfassend

(a) das Umsetzen einer Verbindung der Formel

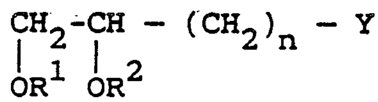

worin $R^1$, $R^2$ und n wie oben definiert sind und Y eine Halogenid- oder p-Toluolsulfonylgruppe ist, mit dem geeigneten Kohlenwasserstoff, ausgewählt aus Chinuclidin, Pyrrolidin, Piperidin oder Morpholin, gegebenenfalls gefolgt von

(b) dem Umsetzen des Produkts von Stufe (a), worin zwei der $R^3$, $R^4$ und $R^5$ zusammengefaßt werden, um Pyrrolidino, Piperidino oder Morpholino zu bilden, mit einem Alkyl- oder Aralkylchlorid, um ein Verbindung der Formel I zu bilden.

3. Verfahren zur Herstellung einer Verbindung der Formel

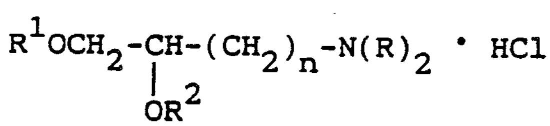

oder eines optischen Isomeren davon, worin $R^1$ und $R^2$ gleich oder verschieden sind und eine Alkyl- oder Alkenylgruppe mit 6 bis 22 Kohlenstoffen sind; jedes R gleich oder verschieden ist und Wasserstoff, Alkyl mit 1 bis 8 Kohlenstoffatomen, Aryl oder Aralkyl mit 7 bis 11 Kohlenstoffen ist oder zwei Gruppen R zusammengefaßt werden, um Pyrrolidino, Piperidino oder Morpholino zu bilden; und n 1—8 ist, umfassend das Umsetzen der freien Base der oben definierten Verbindung mit Chlorwasserstoffsäure.

4. Verfahren nach Anspruch 1 oder Anspruch 3, worin $R^1$ und $R^2$ gleich sind und Alkenyl mit 10 bis 20 Kohlenstoffatomen sind, $R^3$, $R^4$ und $R^5$ Wasserstoff, Methyl oder Ethyl sind und n 1 bis 4 ist.

5. Verfahren nach Anspruch 4, worin eine Verbindung hergestellt wird, die ausgewählt ist aus

(±) N-(2,3-Di-(9-(Z)-octadecenyloxy))-prop-1-yl-N,N-trimethylammoniumchlorid oder einem optischen Isomer davon;

(±) N-(2,3-Di-(4-(Z)-decenyloxy))-prop-1-yl-1-trimethylammoniumchlorid oder einem optischen Isomer davon;

(±) N-(3,4-Di-(9-(Z)-octadecenyloxy))-but-1-yl-N,N,N-trimethylammoniumchlorid oder einem optischen Isomer davon; und

(±) N-(5,6-Di-(9-(Z)-octadecenyloxy))-hex-1-yl-N,N,N-trimethylammoniumchlorid oder einem optischen Isomer davon.

6. Verfahren nach Anspruch 1 oder Anspruch 3, worin $R^1$ und $R^2$ gleich sind und Alkyl mit 10 bis 20

Kohlenstoffatomen sind, $R^3$, $R^4$ und $R^5$ Wasserstoff, Methyl oder Ethyl sind, n 1 bis 4 ist und X ein Halogenidion ist.

7. Verfahren nach Anspruch 6, worin eine Verbindung hergestellt wird, die ausgewählt ist aus

(±) N-(2,3-Di-hexadecyloxy)prop-1-yl-N,N,N-trimethylammoniumchlorid oder einem optischen Isomer davon; und

(±) N-(2,3-Di-octadecyloxy)prop-1-yl-N,N,N-trimethylammoniumchlorid.

8. Verfahren nach Anspruch 1 oder Anspruch 3, worin $R^1$ Alkyl oder Alkenyl mit 14 bis 22 Kohlenstoffatomen ist; $R^2$ Alkyl oder Alkenyl mit 6 bis 14 Kohlenstoffatomen ist, $R^3$, $R^4$ und $R^5$ Wasserstoff, Methyl oder Ethyl sind; n 1 bis 4 ist und X ein Halogenidion ist.

9. Verfahren nach Anspruch 8, worin eine Verbindung hergestellt wird, die ausgewählt ist aus

(±) N-(2-Hexadecyloxy-3-decyloxy)-prop-1-yl-N,N,N-trimethylammoniumchlorid oder einem optischen Isomer davon; und

(±) N-(2-Hexadecyloxy-3-decyloxy)-prop-1-yl-N,N-dimethylaminhydrochlorid.

10. Verwendung einer Verbindung der Formel I, wie in irgendeinem der vorangehenden Ansprüche definiert, bei der Herstellung einer Liposom-Formulierung, umfassend 0,05 bis 10 Gewichtsprozent eines Arzneistoffes, 1 bis 20 Gewichtsprozent einer oberflächenaktiven Komponente, wobei von diesem Gewicht eine Verbindung der Formel I oder irgendeines der vorangehenden Ansprüche 1 bis 100% dieser oberflächenaktiven Komponente umfaßt, und eine wäßrige Lösung in einer Menge, die genügt, 100 Volumenprozent auszumachen.

11. Verwendung einer Verbindung der Formel I, wie in irgendeinem der Ansprüche 1 bis 9 definiert, bei der Herstellung einer pharmazeutischen Formulierung, umfassend 0,05 bis 10 Gewichtsprozent eines Arzneistoffes, 1 bis 20 Gewichtsprozent einer oberflkächenaktiven Komponente, wobei von diesem Gewicht eine Verbindung der Formel I oder irgendeines der Ansprüche 1 bis 7 1 bis 100% dieser oberflächenaktiven Komponente umfaßt, und eine wäßrige Lösung in einer Menge, die genügt, um 100 Volumenprozent auszumachen.

12. Verfahren nach Anspruch 11, worin der Arzneistoff 9-(1,3-Dihydroxy-2-propoxymethyl)-guanidipalmitat, 6α,9α-Difluor-11β,16α,17α,21-tetrahydroxy-pregna-1,4-dien-3,20-dion-16,17-acetonid (Fluocinolonacetonid), 6α,9α-Difluoro-11β,16α,17α,21-tetrahydroxy-pregna-1,4-dien-3,20-dion-16,17-acetonid-21-acetat (Fluocinonid), 1-[4-(4-Chlorphenyl)-2-(2,6-dichlorophenylthio)-n-butyl]imidazolnitrat (Butaconazolnitrat), beta-Interferon, gamma-Interferon, 6-O-Stearoyl-N-acetylmuramyl-L-α-aminobutyryl-D-isoglutamin oder ein Herpesantigen ist.

13. Verfahren nach Anspruch 11 oder Anspruch 12, worin der Arzneistoff 1 bis 5% umfaßt und Formel I 50% oder mehr der oberflächenaktiven Verbindung umfaßt.

14. Verfahren nach Anspruch 11, 12 oder 13, worin in der Verbindung der Formel I $R^1$ und $R^2$ gleich sind und Alkyl oder Alkenyl mit 10 bis 20 Kohlenstoffatomen sind, $R^3$, $R^4$ und $R^5$ Wasserstoff, Methyl oder Ethyl sind, n 1 bis 4 ist und X ein Halogenidion ist.

15. Verfahren nach Anspruch 14, worin die Verbindung der Formel I ausgewählt ist aus

(±) N-(2,3-Di-hexadecyloxy)-prop-1-yl-N,N,N-trimethylammoniumchlorid oder einem optischen Isomer davon;

(±) N-(2,3-Di-octadecyloxy)-prop-1-yl-N,N,N-trimethylammoniumchlorid oder einem optischen Isomer davon; und

(±) N-(2,3-Di-(9-(Z)-octadecenyloxy))-prop-1-yl-N,N,N-trimethylammoniumchlorid oder einem optischen Isomer davon.

16. Verfahren nach Anspruch 11, 12 oder 13, worin in der Verbindung der Formel I $R^1$ und $R^2$ gleich sind und Alkyl mit 10 bis 20 Kohlenstoffatomen oder Alkenyl mit 10 bis 20 Kohlenstoffatomen sind und $R^3$, $R^4$ und $R^5$ Wasserstoff, Methyl oder Ethyl sind und n 1 bis 4 ist.

17. Verfahren nach Anspruch 11, 12 oder 13, worin in der Verbindung der Formel I $R^1$ Alkyl oder Alkenyl mit 14 bis 22 Kohlenstoffatomen ist und $R^2$ eine verschiedene Alkylgruppe mit 6 bis 14 Kohlenstoffatomen ist; $R^3$, $R^4$ und $R^5$ Wasserstoff, Methyl oder Ethyl sind; n 1 bis 4 ist; und X ein Halogenidion ist.

18. Verfahren zur Herstellung einer Verbindung der Formel I, worin n 1 ist, einschließend die reduktive Aminierung einer Verbindung der Formel III

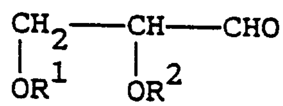

III

worin $R^1$ und $R^2$ wie oben definiert sind, um eine Verbindung der Formel

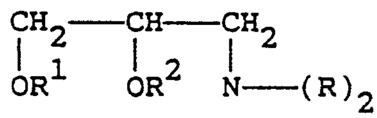

zu bilden, worin R definiert ist, wie $R^3$, $R^4$ und $R^5$ oben definiert sind.

19. Verfahren nach Anspruch 18, worin die Verbindung der Formel III aus einem D-Mannitolderivat der Formel IV

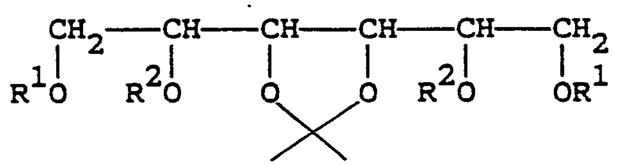

IV

hergestellt wird.

**Revendications pour les Etats contractants: BE CH DE FR GB IT LI LU NL SE**

1. Composé de formule

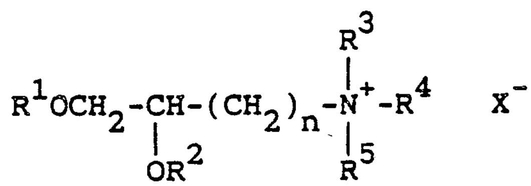

I

ou un isomère optique de ce composé, formule dans laquelle $R^1$ et $R^2$ sont identiques ou différents et représentent un groupe alkyle ou alcényle de 6 à 22 atomes de carbone; $R^3$, $R^4$ et $R^5$ sont identiques ou différents et représentent l'hydrogène, des groupes alkyle ayant 1 à 8 atomes de carbone, aryle, aralkyle de 7 à 11 atomes de carbone ou bien deux ou trois des groupes $R^3$, $R^4$ et $R^5$ forment conjointement un groupe quinuclidino, pyrrolidino, pipéridino ou morpholino; n a une valeur de 1 à 8; et X est un anion pharmaceutiquement acceptable.

2. Composé suivant la revendication 1, dans lequel $R^1$ et $R^2$ sont identiques et représentent des groupes alcényle de 10 à 20 atomes de carbone, $R^3$, $R^4$ et $R^5$ représentent l'hydrogène, le groupe méthyle ou le groupe éthyle et n a une valeur de 1 à 4.

3. Composé suivant la revendication 2, choisi entre

le chlorure de (±) N-(2,3-di-(9-(Z)-octadécényloxy))-prop-1-yl-N,N-triméthylammonium ou l'un de ses isomères optiques;

le chlorure de (±) N-(2,3-di-(4-(Z)-décényloxy))-prop-1-yl-1-triméthylammonium ou l'un de ses isomères optiques;

le chlorure de (±) N-(3,4-di-(9-(Z)-octadécényloxy))-but-1-yl-N,N,N-triméthylammonium ou l'un de ses isomères optiques; et

le chlorure de (±) N-(5,6-di-(9-(Z)-octadécényloxy))-hex-1-yl-N,N,N-triméthylammonium ou l'un de ses isomères optiques.

4. Composé suivant la revendication 1, dans lequel $R^1$ et $R^2$ sont identiques et sont des groupes alkyle de 10 à 20 atomes de carbone, $R^3$, $R^4$ et $R^5$ représentent l'hydrogène, le groupe méthyle ou le groupe éthyle, n a une valeur de 1 à 4 et X est un ion halogénure.

5. Composé suivant la revendication 4, choisi entre

le chlorure de (±) N-(2,3-di-hexadécyloxy)prop-1-yl-N,N,N-triméthylammonium ou l'un de ses isomères optiques; et

le chlorure de (±) N-(2,3-di-octadécyloxy)-prop-1-yl-N,N,N-triméthylammonium.

6. Composé suivant la revendication 1, dans lequel $R^1$ est un groupe alkyle ou alcényle de 14 à 22 atomes de carbone; $R^2$ est un groupe alkyle ou alcényle de 6 à 14 atomes de carbone, $R^3$, $R^4$ et $R^5$ représentent l'hydrogène, un groupe méthyle ou éthyle; n a une valeur de 1 à 4 et X est un ion halogénure.

7. Composé suivant la revendication 6, choisi entre

le chlorure de (±) N-(2-héxadécyloxy-3-décyloxy)-prop-1-yl-N,N,N-triméthylammonium ou l'un de ses isomères optiques; et

le chlorhydrate de (±) N-(2-hexadécyloxy-3-décyloxy)-prop-1-yl-N,N-diméthylamine.

8. Formulation de liposomes, qui comprend 0,05 à 10% en poids d'un médicament, 1 à 20% en poids de composant tensio-actif constitué, en proportion de 1 à 100%, d'un composé de formule I suivant l'une quelconque des revendications précédentes, et une solution aqueuse en quantité suffisante pour représenter 100% en volume.

9. Formulation pharmaceutique, qui comprend 0,05 à 10% en poids d'un médicament, 1 à 20% d'un composant tensio-actif dont une proportion de 1 à 100% est constituée d'un composé de formule I suivant l'une quelconque des revendications 1 à 7, et une solution aqueuse en quantité suffisante pour représenter 100% en volume.

10. Formulation suivant la revendication 9, dans laquelle ledit médicament est le dipalmitate de 9-(1,3-dihydroxy-2-propoxyméthyl)guanine, le 6α,9α-difluoro-11β,16α,17α,21-tétrahydroxy-prégna-1,4-diène-3,20-dione-16,17-acétonide (acétonide de fluocinolone), le 21-acétate de 6α,9α-difluoro-11β,16α,17α,21-tétrahydroxy-prégna-1,4-diène-3,20-dione-16,17-acétonide(fluocinonide), le nitrate de 1-[4-(4-chlorophényl)-2-(2,6-dichlorophénylthio)-n-butyl]imidazole (nitrate de butaconazole), le bêta-interféron, le gamma-interféron, la 6-O-stéaroyl-N-acétylmuramyl-L-α-aminobutyryl-D-isoglutamine ou un antigène de l'herpès.

11. Formulation suivant la revendication 9 ou la revendication 10, dans laquelle le médicament constitue une proportion de 1 à 5% et le composé de formule I constitue 50% ou plus de 50% du composant tensio-actif.

12. Formulation suivant la revendication 9, 10 ou 11, contenant un composé de formule I dans laquelle $R^1$ et $R^2$ sont identiques et sont des groupes alkyle ou alcényle de 10 à 20 atomes de carbone, $R^3$, $R^4$ et $R^5$ représentent l'hydrogène, un groupe méthyle ou éthyle, n a une valeur de 1 à 4 et X est un ion halogénure.

13. Formulation suivant la revendication 12, contenant un composé de formule I qui est choisi entre

le chlorure de (±) N-(2,3-di-hexadécyloxy)-prop-1-yl-N,N,N-triméthylammonium ou l'un de ses isomères optiques;

le chlorure de (±) N-(2,3-di-octadécyloxy)-prop-1-yl-N,N,N-triméthylammonium ou l'un de ses isomères optiques; et

le chlorure de (±) N-(2,3-di-(9-(Z)-octadécényloxy))-prop-1-yl-N,N,N-triméthylammonium ou l'un de ses isomères optiques.

14. Formulation suivant la revendication 9, 10 ou 11, contenant un composé de formule I dans laquelle $R^1$ et $R^2$ sont identiques et sont des groupes alkyle de 10 à 20 atomes de carbone ou alcényle de 10 à 20 atomes de carbone et $R^3$, $R^4$ et $R^5$ représetent l'hydrogène, le groupe méthyle ou éthyle et n a une valeur de 1 à 4.

15. Formulation suivant la revendication 9, 10 ou 11, dans laquelle $R^1$ est un groupe alkyle ou alcényle de 14 à 22 atomes de carbone et $R^2$ est un groupe alkyle différent ayant 6 à 14 atomes de carbone; $R^3$, $R^4$ et $R^5$ représentent l'hydrogène, un groupe méthyle ou éthyle; n a une valeur de 1 à 4; et X est un ion halogénure.

16. Procédé de préparation d'un composé suivant l'une quelconque des revendications 1 à 7, qui consiste à faire réagir un composé de formule:

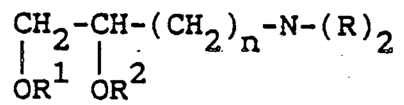

dans laquelle $R^1$, $R^2$ et n sont tels que définis ci-dessus et chaque groupe R a la même définition que $R^3$, $R^4$ et $R^5$ ci-dessus, avec un chlorure d'alkyle ou d'aralkyle pour former un composé de formule I.

17. Procédé de préparation d'un composé de formule:

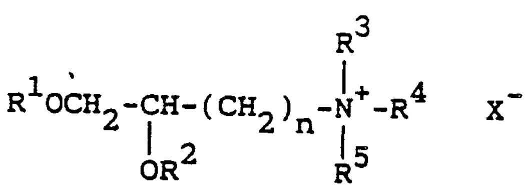

I

ou d'un isomère optique de ce composé, formule dans laquelle $R^1$ et $R^2$ sont identiques ou différents et représentent un groupe alkyle ou alcényle de 6 à 22 atomes de carbone; aucun des groupes $R^3$, $R^4$ et $R^5$ ou l'un de ces groupes représente l'hydrogène, un groupe alkyle ayant 1 à 8 atomes de carbone, aryle ou aralkyle de 7 à 11 atomes de carbone et deux ou trois de $R^3$, $R^4$ et $R^5$ forment conjointement un groupe quinuclidino, pyrrolidino, pipéridino ou morpholino; n a une valeur de 1 à 8; et X est un anion pharmaceutiquement acceptable, procédé qui consiste

(a) à faire réagir un composé de formule

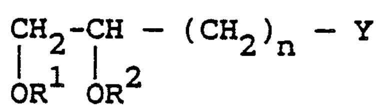

dans laquelle $R^1$, $R^2$ et n sont tels que définis ci-dessus et Y est un groupe halogénure ou p-toluène-sulfonyle, avec l'hydrocarbure approprié choisi entre la quinuclidine, la pyrrolidine, la pipéridine ou la morpholine, la réaction étant facultativement suivie par

(b) la réaction du produit de l'étape (a) dans lequel deux des groupes $R^3$, $R^4$ et $R^5$ forment conjointement un groupe pyrrolidino, pipéridino ou morpholino, avec un chlorure d'alkyle ou d'aralkyle pour former un composé de formule I.

18. Composé de formule

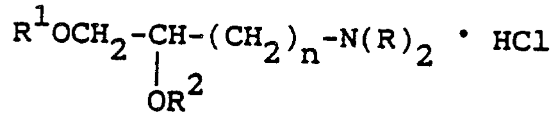

ou l'un de ses isomères optiques, formule dans laquelle $R^1$ et $R^2$ sont identiques ou différents et représentent un groupe alkyle ou alcényle de 6 à 22 atomes de carbone; les groupes R sont identiques ou différents et représentent chacun l'hydrogène, un groupe alkyle de 1 à 8 atomes de carbone, aryle ou aralkyle de 7 à 11 atomes de carbone, ou bien deux groupes R forment conjointement un noyau pyrrolidino, pipéridino ou morpholino; et n a une valeur de 1 à 8.

19. Procédé de préparation d'un composé de formule I dans laquelle n est égal à 1, procédé qui comprend l'amination par voie de réduction d'un composé de formule III

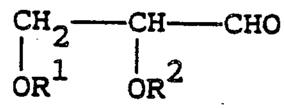

III

dans laquelle $R^1$ et $R^2$ sont tels que définis ci-dessus, pour former un composé de formule

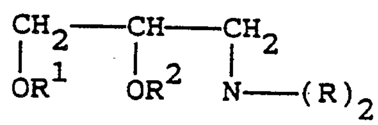

dans laquelle R a la même définition que $R^3$, $R^4$ et $R^5$ ci-dessus.

20. Procédé suivant la revendication 19, dans lequel le composé de formule III est produit à partir d'un dérivé de D-mannitol de formule IV

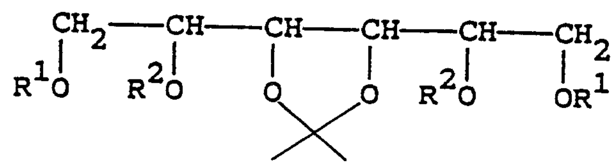

IV

**Revendications pour l'Etat contractant: AT**

1. Procédé de préparation d'un composé de formule

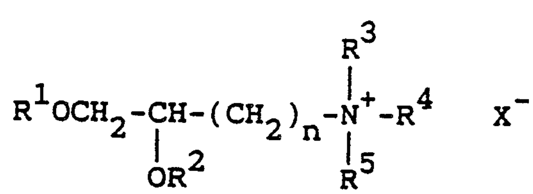

I

ou d'un isomère optique de ce composé, formule dans laquelle $R^1$ et $R^2$ sont identiques ou différents et représentent un groupe alkyle ou alcényle de 6 à 22 atomes de carbone; $R^3$, $R^4$ et $R^5$ sont identiques ou différents et représentent l'hydrogène, des groupes alkyle ayant 1 à 8 atomes de carbone, aryle, aralkyle de 7 à 11 atomes de carbone ou bien deux ou trois des groupes $R^3$, $R^4$ et $R^5$ forment conjointement un groupe quinuclidino, pyrrolidino, pipéridino ou morpholino; n a une valeur de 1 à 8; et X est un anion pharmaceutiquement acceptable, qui consiste à faire réagir un composé de formule

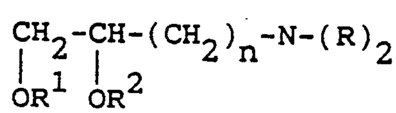

dans laquelle $R^1$, $R^2$ et n ont les définitions données ci-dessus et chaque groupe R est défini comme $R^3$, $R^4$ et $R^5$ ci-dessus, avec un chlorure d'alkyle ou d'aralkyle pour former un composé de formule I.

2. Procédé de préparation d'un composé de formule

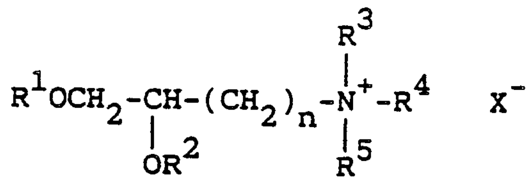

I

ou d'un isomère optique de ce composé, formule dans laquelle $R^1$ et $R^2$ sont identiques ou différents et représentent un groupe alkyle ou alcényle de 6 à 22 atomes de carbone; aucun des groupes $R^3$, $R^4$ et $R^5$ ou l'un de ces groupes représente l'hydrogène, un groupe alkyle ayant 1 à 8 atomes de carbone, aryle ou aralkyle de 7 à 11 atomes de carbone et deux ou trois de $R^3$, $R^4$ et $R^5$ forment conjointement un groupe quinuclidino, pyrrolidino, pipéridino ou morpholino; n a une valeur de 1 à 8; et X est un anion pharmaceutiquement acceptable, procédé qui consiste

(a) à faire réagir un composé de formule

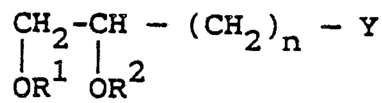

dans laquelle $R^1$, $R^2$ et n sont tels que définis ci-dessus et Y est un groupe halogénure ou p-toluène-sulfonyle, avec l'hydrocarbure approprié choisi entre la quinuclidine, la pyrrolidine, la pipéridine ou la morpholine, la réaction étant facultativement suivie par

(b) la réaction du produit de l'étape (a) dans lequel deux des groupes $R^3$, $R^4$ et $R^5$ forment conjointement un groupe pyrrolidino, pipéridino ou morpholino, avec un chlorure d'alkyle ou d'aralkyle pour former un composé de formule I.

3. Procédé de préparation d'un composé de formule

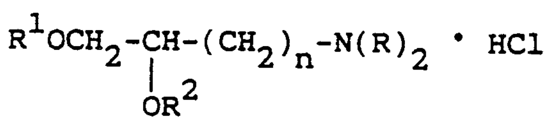

ou de l'un de ses isomères optiques, formule dans laquelle $R^1$ et $R^2$ sont identiques ou différents et représentent un groupe alkyle ou alcényle de 6 à 22 atomes de carbone; chacun des groupes R qui sont identiques ou différents représentent l'hydrogène, un groupe alkyle de 1 à 8 atomes de carbone, aryle ou aralkyle de 7 à 11 atomes de carbone ou bien deux groupes R forment conjointement un noyau pyrrolidino, pipéridino ou morpholino; et n a une valeur de 1 à 8, procédé qui consiste à faire réagir la base libre du compose défini ci-dessus avec l'acide chlorhydrique.

4. Procédé suivant la revendication 1 ou la revendication 3, dans lequel $R^1$ et $R^2$ sont identiques et sont des groupes alcényle de 10 à 20 atomes de carbone, $R^3$, $R^4$ et $R^5$ représentent l'hydrogène, un groupe méthyle ou éthyle et n a une valeur de 1 à 4.

5. Procédé suivant la revendication 4, dans lequel est préparé un composé choisi entre

le chlorure de (±) N-(2,3-di-(9-(Z)-octadécényloxy))-prop-1-yl-N,N-triméthylammonium ou l'un de ses isomères optiques;

le chlorure de (±) N-(2,3-di-(4-(Z)-décényloxy))-prop-1-yl-1-tri-méthylammonium ou l'un de ses isomères optiques;

le chlorure de (±) N-(3,4-di-(9-(Z)-octadécényloxy))-but-1-yl-N,N,N-triméthylammonium ou l'un de ses isomères optiques; et

le chlorure de (±) N-(5,6-di-(9-(Z)-octadécényloxy))-hex-1-yl-N,N,N-triméthylammonium ou l'un de ses isomères optiques.

6. Procédé suivant la revendication 1 ou la revendication 3, dans lequel $R^1$ et $R^2$ sont identiques et sont des groupes alkyle de 10 à 20 atomes de carbone, $R^3$, $R^4$ et $R^5$ représentent l'hydrogène, le groupe méthyle ou le groupe éthyle, n a une valeur de 1 à 4 et X est un ion halogénure.

7. Procédé suivant la revendication 6, dans lequel est préparé un composé choisi entre

le chlorure de (±) N-(2,3-di-hexadécyloxy)prop-1-yl-N,N,N-triméthylammonium ou l'un de ses isomères optiques; et

le chlorure de (±) N-(2,3-di-octadécyloxy)-prop-1-yl-N,N,N-triméthylammonium.

8. Procédé suivant la revendication 1 ou la revendication 3, dans lequel $R^1$ est un groupe alkyle ou alcényle de 14 à 22 atomes de carbone; $R^2$ est un groupe alkyle ou alcényle de 6 à 14 atomes de carbone, $R^3$, $R^4$ et $R^5$ représentent l'hydrogène, le groupe méthyle ou le groupe éthyle; n a une valeur de 1 à 4 et X est un

ion halogénure.

9. Procédé suivant la revendication 8, dans lequel est préparé un composé choisi entre

le chlorure de (±) N-(2-héxadécyloxy-3-décyloxy)-prop-1-yl-N,N,N-triméthylammonium ou l'un de ses isomères optiques; et

le chlorhydrate de (±) N-(2-hexadécyloxy-3-décyloxy)-1-yl-N,N-diméthylamine.

10. Utilisastion d'un composé de formule I suivant l'une quelconque des revendications précédentes dans la préparation d'une formulation de liposomes, qui comprend 0,05 à 10% en poids d'un médicament, 1 à 20% en poids de composant tensio-actif constitué, en proportion de 1 à 100% d'un composé de formule I suivant l'une quelconque des revendications précédentes, et une solution aqueuse en quantité suffisante pour représenter 100% en volume.

11. Utilisation d'un composé de formule I tel que défini dans l'une quelconque des revendications 1 à 9 dans la préparation d'une formulation pharmaceutique qui comprend 0,05 à 10% en poids d'un médicament, 1 à 20% d'un composant tensio-actif dont une proportion de 1 à 100% est constituée d'un composé de formule I suivant l'une quelconque des revendications 1 à 7, et une solution aqueuse en quantité suffisante pour représenter 100% en volume.

12. Procédé suivant la revendication 11, dans lequel le médicament est le dipalmitate de 9-(1,3-dihydroxy-2-propoxyméthyl)guanine, le 6α,9α-difluoro-11β,16α,17α,21-tétrahydroxy-prégna-1,4-diène-3,20-dione-16,17-acétonide (acétonide de fluocinolone), le 21-acétate de 6α,9α-difluoro-11β,16α,17α,21-tétrahydroxy-prégna-1,4-diène-3,20-dione-16,17-acétonide (fluocinonide), le nitrate de 1-[4-(4-chloro-phényl)-2-(2,6-dichlorophénylthio)-n-butyl]imidazole (nitrate de butaconazole), le bêta-interféron, le gamma-interféron, la 6-O-stéaroyl-N-acétylmuramyl-L-α-aminobutyryl-D-isoglutamine ou un antigène de l'herpès.

13. Procédé suivant la revendication 11 ou la revendication 12, dans lequel le médicament est en proportion de 1 à 5% et le composé de formule I constitue 50% ou plus de 50% du composant tensio-actif.

14. Procédé suivant la revendication 11, 12 ou 13, dans lequel $R^1$ et $R^2$ dans le composé de formule I sont identiques et sont des groupes alkyle ou alcényle de 10 à 20 atomes de carbone, $R^3$, $R^4$ et $R^5$ représentent l'hydrogène, un groupe méthyle ou éthyle, n a une valeur de 1 à 4 et X est un ion halogénure.

15. Procédé suivant la revendication 14, dans lequel le composé de formule I est choisi entre

le chlorure de (±) N-(2,3-di-hexadécyloxy)-prop-1-yl-N,N,N-triméthylammonium ou l'un de ses isomères optiques;

le chlorure de (±) N-(2,3-di-octadécyloxy)-prop-1-yl-N,N,N-triméthylammonium ou l'un de ses isomères optiques; et

le chlorure de (±) N-(2,3-di-(9-(Z)-octadécényloxy))-prop-1-yl-N,N,N-triméthylammonium ou l'un de ses isomères optiques.

16. Procédé suivant la revendication 11, 12 ou 13, dans lequel $R^1$ et $R^2$, dans le composé de formule I, sont identiques et sont des groupes alkyle de 10 à 20 atomes de carbone ou des groupes alcényle de 10 à 20 atomes de carbone est $R^3$, $R^4$ et $R^5$ représentent l'hydrogène, un groupe méthyle ou éthyle, et n a une valeur de 1 à 4.

17. Procédé suivant la revendication 11, 12 ou 13, dans lequel $R^1$ dans le composé de formule I est un groupe alkyle ou alcényle de 14 à 22 atomes de carbone et $R^2$ est un groupe alkyle différent, de 6 à 14 atomes de carbone; $R^3$, $R^4$ et $R^5$ représentent l'hydrogène, un groupe méthyle ou éthyle; n a une valeur de 1 à 4; et X est un ion halogénure.

18. Procédé de préparation d'un composé de formule I dans lequel n est égal à 1, procédé qui comprend une amination par voie de réduction d'un composé de formule III

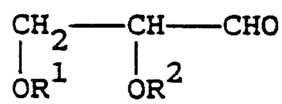

III

dans laquelle $R^1$ et $R^2$ sont tels que définis ci-dessus, pour former un composé de formule

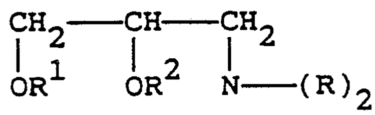

dans laquelle R a la même définition que $R^3$, $R^4$ et $R^5$ ci-dessus.

19. Procédé suivant la revendication 18, dans lequel le composé de formule III est produit à partir d'un dérivé de D-mannitol de formule IV

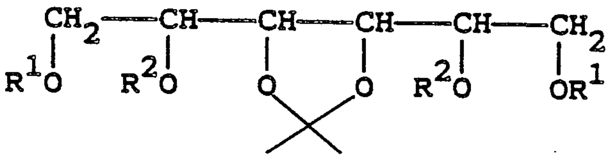

IV